# EUROPÄISCHE PATENTSCHRIFT

(11) **EP 1 539 030 B1**
(45) Veröffentlichungstag und Bekanntmachung des Hinweises auf die Patenterteilung: **11.01.2012**
(21) Anmeldenummer: 03750525.2
(22) Anmeldetag: 11.09.2003
(51) Int. Cl.: A61F 2/01, A61B 17/22

(54) **EXTRAKTIONSVORRICHTUNG**
EXTRACTION DEVICE
DISPOSITIF D'EXTRACTION

(30) Priorität: 11.09.2002 DE 10242444
(43) Veröffentlichungstag der Anmeldung: 15.06.2005
(73) Patentinhaber: PFM Produkte für die Medizin Aktiengesellschaft, 50996 Köln (DE)
(72) Erfinder: FREUDENTHAL, Franz, La Paz (BO); GÜNTHER, Rolf W., 52074 Aachen (DE); SCHMITZ-RODE, Thomas, 52070 Aachen (DE)
(74) Vertreter: Polypatent
(86) Internationale Anmeldenummer: PCT/EP2003/010116
(87) Internationale Veröffentlichungsnummer: WO 2004/026143

(56) Entgegenhaltungen:
- WO-A-00/67664
- WO-A-01/45590
- DE-A- 3 542 667
- US-A1- 2002 082 639

## Beschreibung

Die Erfindung betrifft eine Extraktionsvorrichtung zur Extraktion von Objekten, insbesondere Thromben, Fremdkörpern etc. aus Hohlräumen eines menschlichen oder tierischen Körpers mit einem ersten und einem zweiten komprimierbaren und expandierbaren Fangkorb, zwischen denen das Objekt einfangbar ist, wobei die Fangkörper gegeneinander verschiebbar und ineinander ziehbar sind.

Auch im Zeitalter der prophylaktischen Antikoagulation stellt die akute Lungenembolie ein häufiges und oft lebensbedrohliches Ereignis dar. Die Standardtherapie besteht bei massiver Lungenembolie in der medikamentösen Thrombolyse mit Streptokinase, Urokinase oder Gewebsplasminogenaktivator. Ziel ist dabei die Rekanalisation des betroffenen Gefäßes. Nach Anwendung dieser Standardtherapien zeigen Kontrollen mit bildgebenden Verfahren, beispielsweise durch Echokardiographie, Angiographie, Computertomographie, lediglich eine geringe Rekanalisation nach Thrombolyse. Trotz Hochdosisthrombolyse verstirbt daher ein Teil der Patienten an Rechtsherzversagen.

Alternativ kann bei kontraindizierter Thrombolyse, beispielsweise intrakranieller Verletzung oder nach Operationen, oder bei fehlgeschlagener Thrombolyse das Embolusmaterial operativ ausgeräumt werden. Der Eingriff unter Einsatz der Herz-Lungen-Maschine ist sehr belastend für den Patienten und mit einer hohen Letalität behaftet.

Es wurden daher verschiedene Vorrichtungen entwickelt, um das Ausräumen und Rekanalisieren der Gefäße zu erleichtern. Von Greenfield wurde 1964 ein mechanisches Instrument mit endoskopartiger Steuerung entwickelt, das pulmonale Emboli über eine venöse Punktionsstelle nicht-operativ extrahiert. Aufgrund seiner umständlichen Handhabung hat sich dieser Saugkatheter nicht durchgesetzt. Von Günther und Schmitz-Rode wurde 1991 ein Hochgeschwindigkeitskathetersystem zum Fragmentieren pulmonaler Emboli entwickelt, das jedoch aufgrund seiner technischen Komplexität und nicht ausreichenden pulmonalen Steuerbarkeit des Katheters sich ebenfalls nicht durchsetzten konnte. Ein von Günther und Schmitz-Rode 1995 entwickelter modifizierter Pigtail-Katheter wird rotierbar im embolischen Verschluss bewegt und bewirkt dadurch eine grobe Fragmentation des Embolusmaterials. Hierbei kann jedoch lediglich das weiche und frische Embolusmaterial fragmentiert werden. Bei festeren organisierten Emboli versagt der modifizierte Pigtail-Katheter. Für den Fall von zentral bis peripher mit Embolusmassen angefüllten Pulmonalarterien besteht wenig Aussicht auf einen Rekanalisierungserfolg durch Fragmentation, da die Fragmente nicht nach peripher abschwimmen können.

Diese Nachteile treten bei der US 2002/0095161 A1 nicht auf. Bei dieser Vorrichtung zur Extraktion von beispielsweise ureteralen Steinen werden diese in einem Korb eingefangen, der eine große Öffnung aufweist, die über die Hälfte der Oberfläche des Korbes einnimmt und durch die Steine und Steinfragmente in das Innere des Korbes eintreten können. Außerdem weist der Korb relativ schmale Öffnungen auf, die zum Zurückhalten der Steine und Steinfragmente geeignet sind. Als nachteilig erweist sich hierbei, dass der Einfangvorgang trotz der Möglichkeit, den Korb zu rotieren, relativ mühsam ist und der einzufangende Stein nicht problemlos in den Korb gelangt.

Ein ähnliches Problem tritt bei der Extraktionsvorrichtung nach der US 5,779,716 auf, bei der ein sackartiger Fangkorb mit einem Bügeldraht an seinem proximalen Ende versehen ist, wobei der Bügeldraht die proximale Öffnung des sackartigen Fangkorbs offen hält, um den Einfangvorgang zu unterstützen.

Im Stand der Technik sind außerdem Extraktionsvorrichtungen mit gewendelten Drähten bekannt, zwischen denen ein Stein oder ein anderer Fremdkörper eingefangen werden kann. Ein Beispiel dafür gibt die WO 99/47054. Hierbei steht zu befürchten, dass der Stein während des Zurückziehens beim Bergungsvorgang wieder aus den Schlingen herausrutscht. Dies gilt auch für die in der WO 01/05311 A1 offenbarte Extraktionsvorrichtung und die in der US 2002/0026203 A1 offenbarte Extraktionsvorrichtung.

Greiferartige Einrichtungen, die den zu bergenden Stein erfassen und während des Bergungsvorganges festhalten, sind ebenfalls im Stand der Technik bekannt, beispielsweise aus der WO 00/54672 A1.

Zum Entfernen von Thromben aus dem Vaskularsystem ist es aus der US 5,419,774 A bekannt, in der Extraktionsvorrichtung an deren distalen Ende eine Kammer vorzusehen, in die der Thrombus durch Unterdruck gezogen wird. In der Kammer befindet sich eine Trennvorrichtung, die den Teil des Thrombus abtrennt, der sich in der Kammer befindet. Ein unter Druck stehendes Fluid wird zugeführt, um den Thrombus und das sich bei diesem sammelnde Blut abzutransportieren. Der Aufbau der Vorrichtung ist jedoch relativ aufwendig, da zum Einen eine Einrichtung zum Aufbringen eines Unterdrucks vorgesehen sein muss und zum Anderen eine Einrichtung zum Zuführen einer unter Druck stehenden Flüssigkeit. Außerdem muss eine Einrichtung zum Trennen des Thrombus vorgesehen sein. Diese Nachteile betreffen auch den Katheter zum Bearbeiten und Entfernen von weichen und harten Substanzen zur Verwendung in der invasiven Mikrochirurgie und Gefäßbehandlung gemäß der DE 197 34 890 C1. Der Anwendungsbereich ist hierbei das Entfernen von beispielsweise Gewebe oder Gallensteinen.

Zum Entfernen von Thromben ist im Stand der Technik außerdem aus der US 2002/0026211 A1 bekannt, eine Vorrichtung und ein Verfahren zum Filtern von Emboli oder Entfernen von Thromben aus einem Gefäß vorzusehen, bei dem die Vorrichtung einen Vaskularfilter zum Ergreifen der Emboli und optional ein Thrombektomie-Element zum Entfernen des Thrombus enthält. Der Vaskularfilter enthält einen Tragring mit einem oder mehreren Gelenkbereichen, die nahe einem distalen Ende eines Führungsdrahtes befestigt sind, sowie ein blutdurchlässiges Säckchen, das an dem Tragring befestigt ist. Der Tragring bildet die Öffnung des blutdurchlässigen Säckchens und hält diese offen. Es können zwei hintereinander angeordnete Säckchen vorgesehen sein, deren Öffnungen in dieselbe Richtung, nämlich nach proximal zeigen. Mit dem ersten Vaskularfilter wird der Thrombus eingefangen und mit dem zweiten die verbleibenden Emboli. Beide Vaskularfilter werden in ein Rohr bzw. einen Katheter zurückgezogen, zusammen mit dem Thrombus und den Emboli. Diese Vorrichtung erweist sich als nachteilig aufgrund der Tragringe in Verbindung mit dem blutdurchlässigen Säckchen, da deren Herstellungsaufwand aufgrund der in den Tragringen vorgesehenen Gelenke recht hoch ist. Außerdem ist die Verletzungsgefahr des Gefäßes, aus dem der Thrombus bzw. die Emboli entfernt werden sollen, aufgrund der Verwendung eines Tragrings recht hoch, da dieser im Vergleich zu dem Gefäß starr und unbeweglich ist und gegebenenfalls an der Gefäßinnenwand schabt.

Eine weitere Vorrichtung zum Entfernen von Thromben ist aus der US 5,011,488 A bekannt. Hierbei enthält ein Vaskularkathetersystem ein äußeres flexibles Rohr, ein inneres flexibles Rohr, das in dem Lumen des äußeren flexiblen Rohres angeordnet ist, einen expandierbaren Körper, der an einem distalen Ende eines dritten flexiblen Rohres angeordnet ist, das seinerseits in dem Lumen des inneren flexiblen Rohres angeordnet ist. Das innere Rohr enthält eine expandierbare Spitze, die sich öffnen kann, um im Wesentlichen den Querschnitt eines Blutgefäßes einzunehmen. Der expandierbare Körper wird durch das Thrombusgebiet hindurch ausgestreckt und expandiert. Das zu entfernende Thrombusmaterial befindet sich dann zwischen den beiden expandierten Teilen, wobei die expandierte Spitze ein aufblasbarer Körper ist, der sich an der Innenwand des Gefäßes anlegt und beim Zurückziehen in das Rohr das Thrombusmaterial von der Gefäßinnenwand zu dem expandierten Körper hin abschabt und mitnimmt. Der aufblasbare Körper oder Ballon passt dabei in den expandierten offenen Körper hinein und ist entsprechend diesem kegelstumpfförmig. Der expandierte Körper weist eine Mehrzahl von Federelementen auf, die nach dem Herausschieben aus dem Rohr zu einem Öffnen der expandierbaren Spitze führen. Aufgrund der Kegelstumpfform wird der expandierte Körper beim Zurückziehen in das Rohr wieder zusammengeschoben. Dasselbe geschieht mit dem dann innen liegenden aufblasbare Körper oder Ballon. Diese Vorrichtung erweist sich als nachteilig, da bei dem Zurückziehen der beiden Körper das sich zwischen diesen befindende Thrombusmaterial insbesondere beim Komprimieren des aufblasbaren Körpers wieder austreten kann, also nicht sicher zwischen dem aufblasbaren Körper und dem expandierbaren offenen Kegelstumpfkörper festgehalten wird.

Aus der WO 00/51505 A1 ist eine Extraktionsvorrichtung mit nur einem ballonartig aufweitbaren distalen Abschnitt bekannt, das verschlungene Drähte aufweist, die mit einem Gewebe belegt sind. Zum Ausräumen eines Gefäßes schabt das aufgeweitete Ende an der Gefäßwand entlang, und das Gewebe verhindert ein Eindringen von abgeschabten Fremdkörpern in die Vorrichtung bzw. den aufgeweiteten Abschnitt.

Die DE 692 28 326 T2 offenbart eine Extraktionsvorrichtung, bei der ein flexibles Schleifenteil mit einem Netz bespannt und an seinem distalen und proximalen Ende auf einem Zugdraht befestigt ist. Das Netz kann durch Verschieben des Schleifenteils entlang dem Zugdraht eine offene und eine geschlossene Form annehmen. Hierdurch kann ein Fremdkörper in dem verschlungenen mit Netz bespannten Schlingenteil eingefangen werden.

Aus der WO 00/53120 A1 ist eine Extraktionsvorrichtung bekannt, bei der zwei Fangkörbe in Ausrichtung der Öffnungen der Fangkörbe zueinander vorgesehen sind. Der distale Fangkorb ist an einem Stab befestigt und innerhalb des anderen Fangkorbs in diesen hineinziehbar angeordnet. Der distale Fangkorb weist ein distales zusammengezogenes Ende auf, wohingegen der proximale Fangkorb ein proximales zusammengezogenes Ende aufweist.

Die US 20021082639 A1 offenbart eine Extraktionsvorrichtung, die zum Einfangen von u.a. Emboli in einem Filter einen zusammenfaltbaren proximal schräg zulaufenden Rahmen aufweist zum Tragen des Filters in einem zusammengefalteten Einführzustand und einem expandierten Zustand. Der schräg zulaufende zusammenfaltbare Rahmen enthält eine Mundöffnung, die so dimensioniert ist, dass sie sich im expandierten Zustand bis an die Gefäßwände heran erstreckt, um in dem expandierten Zustand den Filter gegenüber dem Gefäß zum Einsammeln von Fremdstoffen, die im Gefäß schwimmen, abzudichten. In einer Ausführungsform weist die Extraktionsvorrichtung zwei ineinander ziehbare Fangkörper auf.

Die DE 35 42 667 A1 offenbart eine Vorrichtung zum Entfernen von Körpersteinen aus Körperhöhlungen, die an einem Endoskop angebracht ist. Im Schaft des Endoskops sind Steuerglieder geführt, an deren inkorporalem Ende eine flexible Schlaufe, an der ein sackförmiger Behälter angebracht ist, vorgesehen ist. Die Steuerglieder sind in einem Führungskanal innerhalb des Schaftes geführt. Betätigungsglieder zum Schließen des sackförmigen Behälters können in gesonderten Führungskanälen im Schaft geführt werden.

Aus der WO 00/67664 A1 ist eine Emboli-Schutzeinrichtung mit einem zusammenfaltbaren Filterelement bekannt, befestigt an einem Träger, wie einem Führungsdraht. Ein Netzfilter ist mit verwobenen Fasern an seinem distalen Ende beschrieben, die in einer Anzahl von Bündeln am proximalen Ende zusammengeführt sind.

Die WO 01/45590 A2 offenbart eine Filtereinrichtung für ein Blutgefäß eines Patienten, angeordnet am Ende eines katheterartigen Schafts. Das Filterelement weist eine Membran auf, die durch Verstrebungen getragen wird, die distal an dem katheterartigen Schaft angesetzt sind. Beim Betätigen gelangen die Verstrebungen in eine expandierte Position und bringen dadurch die Membran in eine Position, die es dieser erlaubt, dass Blut durch das Filterelement hindurchfließen kann, wobei Emboli in der Membran eingefangen werden. Das embolisierende Material wird entweder durch Absaugen oder durch Flachlegen der Verstrebungen und Einfangen des embolisierenden Materials innerhalb des Verstrebungssystems zum Entfernen zusammen mit dem Filterelement entfernt.

Der vorliegenden Erfindung liegt nun die Aufgabe zugrunde, die vorstehend genannten Nachteile des Standes der Technik zu vermeiden und eine Extraktionsvorrichtung zur Extraktion von Objekten, insbesondere Thromben, Fremdkörpern etc. aus Hohlräumen eines menschlichen oder tierischen Körpers zu schaffen, bei der eine sichere Ummantelung des Objektes stattfindet, wodurch dieses gefahrlos geborgen und aus dem Hohlraum des menschlichen oder tierischen Körpers entfernt werden kann. Insbesondere soll eine effektive nichtoperative Rekanalisation bei teilorganisierten Emboli und bei ausgedehnter Embolisierung der Lungenstrombahn ermöglicht werden.

Die Aufgabe wird durch eine Extraktionsvorrichtung nach dem Oberbegriff des Anspruchs 1 dadurch gelöst, dass zumindest der eine Fangkorb im expandierten Zustand schirmartig und mit flexiblen drahtartigen Verstellelementen zum gezielten Ändern der Form und/oder Position des Fangkorbs so ausgebildet ist, dass der zumindest eine Fangkorb beim Aufweiten in seiner Längserstreckung verkürzt und beim Reduzieren seines Querschnitts verlängert wird, und dass das Objekt in diesem einfangbar und in den anderen Fangkorb hineinziehbar ist. Die Aufgabe wird außerdem für eine Extraktionsvorrichtung nach dem Oberbegriff des Anspruchs 4 dadurch gelöst, dass zumindest ein drahtartiges flexibles Verstellelement an dem distalen und/oder proximalen Ende so befestigt ist, dass der zumindest eine Fangkorb durch diese gezielt dirigierbar und in seiner Form so veränderbar ist, dass der zumindest eine Fangkorb beim Aufweiten in seiner Längserstreckung verkürzt und beim Reduzieren seines Querschnitts verlängert wird. Weiterbildungen der Erfindung sind in den abhängigen Ansprüche definiert.

Dadurch wird eine Extraktionsvorrichtung zur Extraktion von Objekten, insbesondere Thromben, Fremdkörpern etc. aus Hohlräumen eines menschlichen oder tierischen Körpers geschaffen, die es erlaubt, insbesondere Emboli in Pulmonalarterien so einzufangen, zu ummanteln und zu komprimieren, dass sie durch die rechte Herzhälfte hindurch gefahrlos geborgen und über den perkutanen venösen Zugangs femoral oder jugulär aus dem Kreislauf entfernt werden können. Aufgrund der Verwendung zumindest eines schirmartigen Fangkorbs, der in den anderen Fangkorb hineingezogen werden kann, wird die Möglichkeit gegeben, dass Thrombuspartikel in den Fangkörben zerquetscht werden, so dass der zu einem hohen Prozentsatz in dem Thrombus vorliegende Flüssigkeitsanteil austreten kann, und lediglich so kleine Thrombuspartikel mit dieser Flüssigkeit in das Blutkreislaufsystem zurückgelangen, dass hierdurch keine vitale Bedrohung mehr auftritt. Die größeren Thrombuspartikel hingegen werden durch ein Schleusenelement aus dem menschlichen oder tierischen Körper herausgezogen, so dass diese ebenfalls keine vitale Bedrohung mehr darstellen. Die Extraktionsvorrichtung ist daher insbesondere als Thrombektomiesystem einsetzbar, wobei insbesondere eine massive Lungenembolie vermieden werden kann. Vaskulare Ablagerungen können festgehalten und entfernt werden, wobei dies insbesondere auch für die Gefäße, die in Richtung Gehirn führen, möglich ist. Ein anderes Anwendungsgebiet ist die Fremdkörperentfernung, zu der beispielsweise auch die Entfernung von Nierensteinen, Gallensteinen sowie sonstigen sich im Körper bildenden Körpern gerechnet wird. Außerdem können "echte" Fremdkörper entfernt werden, beispielsweise aus dem Lungensystem, aus dem gastrointestinalen System und allgemein aus allen Hohlräumen eines menschlichen oder tierischen Körpers. Die Extraktionsvorrichtung ist somit nicht nur für den Einsatz in allen Gefäßregionen ausgebildet, beispielsweise in miniaturisierter Form zur Thrombektomie in Hämodialyse-Shunts, bei der minimal invasiven Chirurgie sowie der Roboter Chirurgieintervention, sondern allgemein zum Entfernen von jeder Art von Fremdkörpern oder Körpern aus Hohlräumen eines menschlichen oder tierischen Körpers, beispielsweise auch durch Laparoskopie.

Durch das Vorsehen einer mit flexiblen drahtartigen Verstellelementen versehenen Schirmform des zumindest einen Fangkorbs kann eine beliebige gezielte Größenverstellung und damit Veränderung des Einfangbereichs erzielt werden. Vorzugsweise öffnet sich der schirmartige Fangkorb zu dem anderen Fangkorb hin. Hierdurch kann das Objekt, z.B. Thrombus, Fremdkörper, etc. zwischen den beiden mit ihren Öffnungen zueinander gerichteten Fangkörben eingefangen und aufgenommen werden. Ein "Entrinnen" des Objekts wird dabei nicht mehr zugelassen. Alternativ kann der schirmartige Fangkorb sich von dem anderen Fangkorb weg weisend öffnen. Diese Möglichkeit erweist sich als vorteilhaft, wenn der eine Fangkorb nicht an dem zu bergenden Objekt vorbeigeführt werden kann, um dieses von der anderen Seite her zu erfassen. Dadurch sind dann beide Fangkörper auf der gleichen Seite, nämlich proximal, von dem Objekt angeordnet, und der schirmartige Fangkorb greift von dieser proximalen Seite an dem Objekt an. Nachfolgend wird dieses aber ebenfalls in den anderen Fangkorb hineingezogen, um es zu bergen und sofern möglich zu komprimieren.

Bevorzugt sind beide Fangkörbe mit zumindest einem Verstellelement zum Verstellen der Form und/oder Position der Fangkörbe versehen. Das zumindest eine Verstellelement dient dabei dem Vor- und Zurückziehen des Fangkorbs in dem Hohlraum, aus dem das Objekt, z.B. Thrombus, Fremdkörper etc. entfernt werden soll und dem Verstellen der Form und Größe des Fangkorbs, um sich dem Objekt und seiner Position besonders gut anpassen zu können. Bevorzugt weist das zumindest eine Verstellelement ein oder mehrere dünne Drähte auf. Durch diese kann eine besonders gute und genaue Formänderung des Fangkorbs herbeigeführt werden. Bei zumindest einem Fangkorb kann ein Betätigen der Verstellelemente zur Aufweitung Veränderung des Fangkorbs führen. Dies erweist sich gerade bei dem Fangkorb als vorteilhaft, in den der andere Fangkorb hineingezogen werden soll. Der andere schirmartige Fangkorb ist vorteilhaft ebenfalls mit Verstellelementen versehen, um ihn so um den Thrombus, Fremdkörper etc. herum zu positionieren, dass dieser sicher eingefangen werden kann.

Dies unterscheidet die erfindungsgemäßen Fangkörbe entscheidend von denen in der US 2002/0095161 A1 offenbarten, da bei diesen nur ein größerer distaler Fangkorb ohne Verstellelemente offenbart als vorteilhaft ist. Bei ebenfalls bevorzugtem Vorsehen einer Führungskanüle, die an dem distalen Ende des Fangkorbs bzw. des ersten Fangkorbs befestigt ist, kann bei proximal vorgesehen Verstellelementen insbesondere die Öffnungsweite dieses ersten Fangkorbes verändert werden. Hierbei kann über die Führungskanüle der Fangkorb an der optimalen Stelle gehalten und über die Verstellelemente so weit wie erforderlich geöffnet werden. Bei einem bevorzugt ebenfalls vorgesehenen Schleusenelement, durch das die Fangkörper bis zu dem Ort, an dem das Objekt. z.B. Thrombus, Fremdkörper etc. in dem Hohlraum sitzen, befördert bzw. vorgeschoben werden können, kann der Fangkorb zusammen mit der Führungskanüle und den Verstellelementen zurückgezogen werden, wobei ein Komprimieren des Fangkorbs bzw. der Fangkörbe stattfindet.

Vorzugsweise ist das zumindest eine Verstellelement auf der Außenseite und/oder Innenseite des zumindest einen Fangkorbs angeordnet. Besonders bevorzugt ist das zumindest eine Verstellelement in die Mantelfläche des Fangkorbs zumindest teilweise integriert und/oder dort eingeflochten. Dies erweist sich insbesondere bei dem zweiten Fangkorb, der den ersten Fangkorb in sich aufnimmt, als vorteilhaft, um diesen gezielt und reproduzierbar in eine optimale Form bringen zu können. Vorzugsweise ist dabei das proximale Ende des Fangkorbes in einem rohrförmigen Element, insbesondere einem Katheter, festgelegt und das zumindest eine Verstellelement durch das rohrförmige Element geführt. Durch Aufbringen einer Kraft in distaler oder proximaler Richtung kann der Fangkorb in seiner Form verstellt werden. Der Fangkorb ist dabei so ausgebildet, dass er beim Aufweiten in seiner Längserstreckung verkürzt und beim Reduzieren seines Querschnitts verlängert wird. Bevorzugt kann der zumindest eine Fangkorb auf einen größeren Durchmesser als den Durchmesser des auszuräumenden Hohlraums, insbesondere Gefäßes, zum partiellen Aufweiten desselben expandiert werden. Aufgrund dieser vorteilhaften Möglichkeit der Verkürzung seiner Längserstreckung kann bei gleichzeitiger Gefäßaufweitung und Positionierung vor einem einzufangenden Objekt dieses von selbst in den Fangkorb hineinspringen. Bei den aus dem Stand der Technik bekannten Stents wird eine solche Verkürzung gerade nicht gewünscht, um Fremdkörper besonders gut in diese hineinbringen zu können. Im Rahmen der vorliegenden Erfindung erweist sich die Querschnittsveränderung und Verkürzung der Längserstreckung des Fangkorbs jedoch für den Einfangvorgang als besonders vorteilhaft.

Zum Verstärken der Verbindung zwischen rohrförmigem Element und Fangkorb kann bevorzugt am proximalen Ende des Fangkorbs ein Hülsenelement vorgesehen sein. Dieses ist jedoch vorteilhaft so aufgebracht, dass ein Verstellen insbesondere der Form des Fangkorbs über die Verstellelemente problemlos möglich ist.

Das zumindest eine Verstellelement ragt bevorzugt über die ausgestreckte Länge des zumindest einen Fangkorbs hinaus und ist vorzugsweise proximal betätigbar angeordnet. Zum Betätigen des oder der Verstellelemente(s) ist vorzugsweise ein Handgriff vorgesehen, wobei durch einen Handgriff bevorzugt die Verstellelemente aller Fangkörper betätigt werden können. Der Handgriff weist hierzu bevorzugt unterschiedliche Betätigungselemente auf, an denen die einzelnen Verstellelemente befestigt werden können.

Vorzugsweise sind das oder die Verstellelement(e) verzweigt am Fangkorb befestigt und proximal in Gruppen zusammengeführt. Dies erweist sich vor allem an dem schirmartigen Fangkorb als vorteilhaft, damit dieser besonders gut um das Objekt, z.B. Thrombus, Fremdkörper etc. herumgeführt und gleichmäßig zu dem Schleusenelement bzw. zweiten Fangkorb zurückgezogen werden kann. Vorzugsweise ist das zumindest eine Verstellelement einteilig mit dem Fangkorb. Hierdurch ist kein Ablösen der Verstellelemente von dem Fangkorb zu befürchten. Außerdem kann sich die Betätigungskraft optimal über die Mantelfläche des Fangkorbs fortsetzen, so dass die Betätigung des zumindest einen Verstellelementes den gewünschten Effekt der Verstellung des Fangkorbs so schnell wie möglich zeigt.

Vorzugsweise ist der Abstand zwischen dem distalen Ende des Fangkorbes und der zumindest einen proximalen Befestigungs- oder Austrittsstelle des zumindest einen Verstellelements für unterschiedliche Ausbildungen des Fangkorbs gleichbleibend. Dies erweist sich beim Einführen in das Schleusenelement als vorteilhaft, da ein Verlieren des eingefangenen Fremdkörpers. Thrombus etc. hierdurch nicht zu befürchten ist. Auch ein Stocken der Einführbewegung in das Element kann dadurch vermieden werden.

Bevorzugt sind quer zu der Längserstreckung des zumindest einen Fangkorbs Reduzierelemente vorgesehen, insbesondere im Bereich der proximalen und/oder distalen Enden des Fangkorbs und/oder im Bereich der zumindest einen proximalen Befestigungs- oder Austrittsstelle des zumindest einen Verstellelements. Besonders bevorzugt sind die Reduzierelemente Schlingen. Hierdurch kann vor dem Einführen in das Schleusenelement bzw. einen Katheter der zumindest eine Fangkorb verschlossen werden, so dass das Objekt vollständig in dem Fangkorb gefangen ist und nicht wieder unbeabsichtigt in den Hohlraum, insbesondere ein Blutgefäß austreten kann. Dies erweist sich besonders für den schirmartigen Fangkorb als vorteilhaft.

Bevorzugt besteht zumindest ein Fangkorb aus einem Geflecht und/oder Gewebe und/oder Gelege, insbesondere einem Drahtgeflecht und/oder Drahtgewebe und/oder Drahtgelege. Besonders bevorzugt ist das zumindest eine Verstellelement aus einem Teil des Geflechts, Gewebes oder Geleges ausgebildet. Bei Vorsehen eines aus einem solchen Geflecht, Gewebe oder Gelege bestehenden Fangkorbes kann durch die Maschengröße die Größe derjenigen Partikel bestimmt werden, die nach dem Komprimieren des Fangkorbes durch Einführen in beispielsweise das Schleusenelement oder einen Katheter wieder in die Blutbahn bzw. allgemein den Hohlraum des menschlichen oder tierischen Körpers austreten können. Ein Ausquetschen oder Auspressen insbesondere eines Thrombus oder Embolus ist dabei besonders gut möglich. Alternativ kann zumindest ein Fangkorb vorteilhaft aus einem zumindest über einen Teil seiner Länge geschlitzten Rohr bestehen. Hierbei wird insbesondere auf die DE 100 00 137 A1 Bezug genommen. Die insbesondere in der Figur 1 dieser Druckschrift dargestellten Elemente können hier vorteilhaft ebenfalls als Fangkörper eingesetzt werden. Es erweist sich als besonders vorteilhaft, wenn der oder die Schnitte in dem geschlitzten Rohr so ausgeführt sind, dass das Verhältnis von Verkürzung und Aufweitung beim Expandieren des insbesondere proximal festgelegten Fangkorbs maximal wird. Besonders bevorzugt sind der oder die Schnitt(e) in dem geschlitzten Rohr lang im Vergleich zu der Längserstreckung des Fangkorbs ausgeführt. Hierdurch kann eine besonders gute Aufweitung und Verkürzung beim Expandieren des Fangkorbs erzeugt werden.

Vorzugsweise ist ein Führungsdraht oder Innenmandrin vorgesehen, entlang dem der zumindest eine oder die beiden Fangkörbe verschiebbar und/oder in den Hohlraum einführbar sind. Hierdurch kann eine optimale Ausrichtung gegenüber dem einzufangenden Objekt (Thrombus, Fremdkörper etc.) vorgesehen werden, insbesondere beim Zurückziehen in das Schleusenelement bzw. einen Katheter.

Bevorzugt weist zumindest der zweite Fangkorb einen selbstöffnenden Teilbereich und einen selbstschließenden Teilbereich auf, wobei der selbstschließende Teilbereich durch zumindest ein Verstellelement gezielt geöffnet werden kann. Ein Festhalten eines eingefangenen Objektes ist dadurch besonders gut und wirkungsvoll möglich.

Die Extraktionsvorrichtung ist vorzugsweise ebenfalls in Verbindung mit einem Endoskop mit oder ohne Vorsehen des Schleusenelements verwendbar. Hierdurch kann der Einfang- und Extraktionsvorgang besonders gut von außen beobachtet werden, da insbesondere nicht nur eine Beobachtungsoptik, sondern auch eine Lichtquelle an den Operationsort mit vorgeschoben werden kann.

Vorzugsweise bestehen Teilbereiche des zumindest einen Fangkorbs aus Material mit unterschiedlichem Durchmesser. Besonders bevorzugt besteht ein expandierbarer Teilbereich des zumindest einen Fangkorbs aus einem Material mit einem dünneren Querschnitt oder es weist bevorzugt ein Geflecht, Gelege oder Gewebe mit Fäden unterschiedlichen Durchmessers und/oder Querschnitts auf. Hierdurch kann ein Teilbereich des Fangkorbs geschaffen werden, der besonders leicht expandiert werden kann, wohingegen der nicht zu expandierende Teilbereich, der insbesondere an dem rohrförmigen Element bzw. Katheter festgelegt ist, aus Material mit einem dickeren Querschnitt bzw. einheitlichen Querschnitt bestehen kann. Hierdurch wird die vorteilhafte Möglichkeit geschaffen, den Fangkorb über seine Mantelfläche hinweg gezielt so auszubilden, dass bestimmte Bereiche besonders gut expandiert werden können, um einen optimalen Einfang- und Extraktionsvorgang zu ermöglichen. Besonders bevorzugt ist das Material des zumindest einen Fangkorbs in zumindest einem Teilbereich chemisch und/oder mechanisch behandelt, insbesondere geätzt, elektropoliert, mikrogeschliffen oder anderweitig behandelt. Zusätzlich oder alternativ kann hierdurch ebenfalls ein Teilbereich mit anderen Expansions- und Kompressionseigenschaften vorgesehen werden als die restlichen Bereiche des Fangkorbes.

Vorzugsweise besteht der zumindest eine Fangkorb aus einem biokompatiblen Material, insbesondere einem Metall oder einer Metalllegierung, insbesondere einem Edelstahl oder Nitinol. Es können auch Teilbereiche des zumindest einen Fangkorbes aus unterschiedlichen Materialien bestehen, die insbesondere unterschiedliche mechanische Eigenschaften aufweisen. Es können also nicht nur Fäden mit unterschiedlichem Durchmesser, sondern auch mit unterschiedlicher Elastizität, Biegsamkeit und/oder mechanischer Belastbarkeit vorgesehen werden.

Vorzugsweise besteht die Führungskanüle und/oder das oder die rohrförmigen Element(e) aus einem biegsamen Material, insbesondere einem Metall, einer Metalllegierung, einem Kunststoff oder einem anderen biegsamen Material oder einer Materialkombination, insbesondere aus Nitinol. Je nach Anwendungsort und Anwendungsform erweist es sich als hilfreich, wenn die Führungskanüle und/oder das oder die rohrförmigen Elemente die Biegungen eines Blutgefäßes oder eines anderen Gefäßes oder Hohlraums nachbilden können, in das oder den sie eingeführt werden. Je nachdem, von wo aus die Extraktionsvorrichtung in den Körper des Patienten eingeführt wird, sind mehr oder weniger Biegungen nachzuvollziehen. Vorteilhaft besteht daher auch das Schleusenelement aus einem stabilen und zumindest teilweise biegsamen Material, insbesondere aus einem Kunststoff, Metall, einer Metalllegierung, insbesondere Nitinol, insbesondere einem dünnwandigen Nitinolrohr. Dieses ist jedoch vorzugsweise so speziell ausgeführt, dass eine Verletzung eines Gefäßes oder anderen Hohlraums, in das oder den das Schleusenelement eingeführt wird, nicht zu befürchten steht. Die Verstellelemente können zum Einen Teil des Geflechts, Gewebes oder Geleges oder aber auch des geschlitzten Rohres des Fangkorbs sein. Das Vorsehen eines geschlitzten Rohres in Form eines Fangkorbs erweist sich insbesondere bei der Reproduzierbarkeit der Form als besonders vorteilhaft, da bei einem Laserschnitt der Rohre eine maschinelle Automatisierung zu einer erheblichen Reduktion des Herstellungspreises führt. Die Verstellelemente bestehen daher vorteilhaft aus dem gleichen Material wie der zumindest eine Fangkorb bzw. die Fangkörbe. Auch das rohrförmige Element kann daher vorzugsweise einteilig mit dem zweiten Fangkorb ausgebildet und zumindest teilweise mit einem ein Expandieren und Komprimieren ermöglichenden Schnitt versehen sein.

Zur näheren Erläuterung der Erfindung werden im Folgenden Ausführungsbeispiele anhand der Zeichnungen näher beschrieben. Diese zeigen in:
Figur 1 eine Draufsicht auf eine erste Ausführungsform einer erfindungsgemäßen Extraktionsvorrichtung,
Figur 2 eine Draufsicht auf eine alternative Ausführungsform eines erfindungsgemäßen zweiten Fangkorbes für eine Extraktionsvorrichtung nach Figur 1,
Figur 3 a)-c) Skizzen dreier Endbereiche von erfindungsgemäß ausgeführten Fangkörben,
Figur 4 eine Draufsicht auf einen ersten erfindungsgemäß ausgeführten Fangkorb mit Führungshülse, Führungsdraht, Verstellelementen, Katheter und Schleusenelement,
Figur 5 eine Draufsicht auf eine zweite Ausführungsform des ersten Fangkorbes mit einzufangendem Objekt,
Figur 6 eine Draufsicht auf eine dritte Ausführungsform eines erfindungsgemäßen ersten Fangkorbes,
Figur 7 eine Draufsicht auf eine vierte Ausführungsform eines erfindungsgemäßen ersten Fangkorbes,
Figur 8 eine Draufsicht auf eine fünfte Ausführungsform eines erfindungsgemäß ausgeführten ersten Fangkorbes,
Figur 9 eine Draufsicht auf eine sechste Ausführungsform eines erfindungsgemäß ausgeführten Fangkorbes mit Reduzierelementen,
Figur 10 eine Draufsicht auf eine siebte Ausführungsform eines ersten Fangkorbes,
Figur 11 eine Draufsicht auf eine alternative Ausführungsform einer erfindungsgemäßen Extraktionsvorrichtung,
Figur 12 eine Draufsicht auf eine weitere alternative Ausführungsform eines erfindungsgemäßen ersten Fangkorbes,
Figur 13 eine Draufsicht auf einen zweiten Fangkorb mit Verstellelementen im komprimierten Zustand,
Figur 14 bis Figur 19 einzelne Schritte des Einfangvorgangs durch den zweiten Fangkorb gemäß der vorliegenden Erfindung,
Figur 20 a) und b) eine Skizze einer weiteren Ausbildungsform eines erfindungsgemäßen Fangkorbes, geschnitten aus einem geschlitzten Rohr in komprimiertem (a) und expandiertem (b) Zustand,
Figur 21 eine perspektivische Ansicht einer alternativen Ausführungsform einer erfindungsgemäßen Extraktionsvorrichtung,
Figur 22 eine Frontalansicht der Extraktionsvorrichtung gemäß Figur 21,
Figur 23 und Figur 24 perspektivische Seitenansichten der Extraktionsvorrichtung gemäß Figur 21 in maximal aus dem Schleusenelement ausgefahrener und in dieses teilweise eingezogener Position,
Figur 25 a)-e) einzelne Schritte des Ablauf einer Bergung pulmonaler Emboli mit einer Extraktionsvorrichtung gemäß Figur 21 in ein Schleusenelement hinein,
Figur 26 eine weitere Ausführungsform der Extraktionsvorrichtung gemäß Figur 21, bei der ein außermittiger Führungsdraht als einstrebiger Fangkorb vorgesehen ist,
Figur 27 eine weitere Ausführungsform einer erfindungsgemäßen Extraktionsvorrichtung mit erstem und zweitem Fangkorb,
Figur 28 eine weitere Ausführungsform einer erfindungsgemäßen Extraktionsvorrichtung mit einer auf die netzartige Struktur des Fangkorbs aufgebrachten Beschichtung,
Figur 29 eine perspektivische Ansicht der mit einer Einrichtung zum Zerteilen in Form einer Drahtes mit aufgefügter Kugel versehenen Extraktionsvorrichtung gemäß Figur 28, und
Figur 30 Detailansichten von verschiedenen Ausführungsvarianten von Drahtenden einer Einrichtung zum Zerteilen.

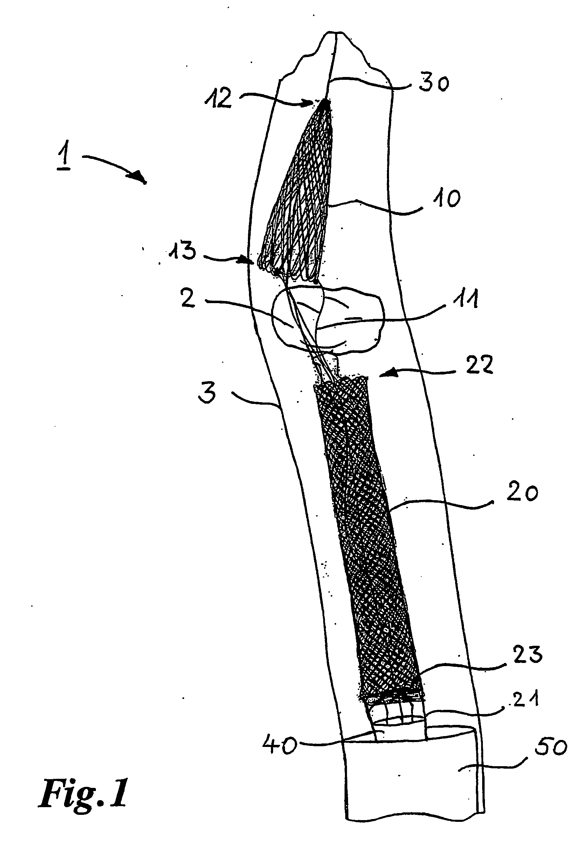

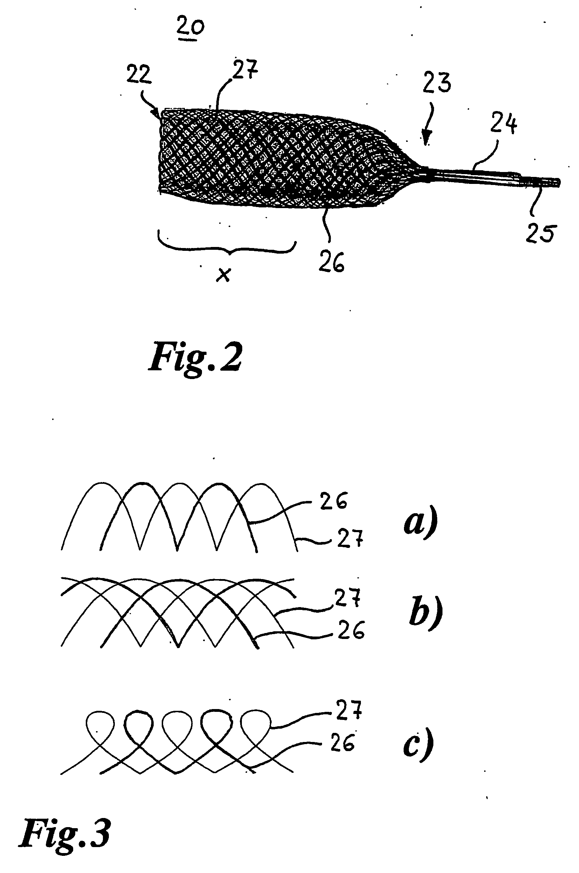

Figur 1 zeigt eine erste Ausführungsform einer erfindungsgemäßen Extraktionsvorrichtung 1 mit einem ersten Fangkorb 10 und einem zweiten Fangkorb 20. Beide Fangkörbe sind entlang einem Führungsdraht 30 geführt. Sie weisen Verstellelemente 11, 21 auf. Die Verstellelemente 11 des ersten Fangkorbes sind ebenso wie der Führungsdraht 30 durch den zweiten Fangkorb 20 hindurchgeführt. Die Verstellelemente 11, sowie der Führungsdraht sind ebenfalls durch einen Katheter 40 hindurchgeführt. Die Verstellelemente 11 können alle oder teilweise innerhalb von diesem verschiebbar bzw. beweglich geführt sein. Vorzugsweise sind die Verstellelemente 21 des zweiten Fangkorbs außerhalb des Katheter geführt, um eine bessere Relativbewegung von erstem und zweiten Fangkorb gegeneinander und vor allem ein Verstellen des zweiten Fangkorbs zu ermöglichen. Der zweite Fangkorb ist dabei am Katheter 40 befestigt, wie in Figur 2 zu sehen.

Erster und zweiter Fangkorb sowie der Katheter 40 können in ein Schleusenelement 50 hineingezogen werden. Das Schleusenelement 50 kann selbst ein Katheter sein. Es wird in einen Hohlraum eines menschlichen oder tierischen Körpers, insbesondere in ein Gefäß hineingeschoben, um die Fangkörbe zu einem Objekt 2 zu bringen, das aus dem Hohlraum 3 entfernt werden soll. Das Objekt kann beispielsweise ein Thrombus, ein Fremdkörper, ein Embolus oder ein anderes Objekt sein, das in einem Gefäß, im Lungensystem, im gastroempathischen System, den Nieren, der Galle oder einem anderen Körperteil bzw. Hohlraum eines menschlichen oder tierischen Körpers sitzt und von dort entfernt werden soll. Um den Weg durch den Körper bzw. die Hohlräume einzuhalten, ist der Führungsdraht 30 vorgesehen. Dieser kann, um eine Verletzung des Hohlraumes, insbesondere Blutgefäßes zu vermeiden, eine atraumatische Spitze aufweisen.

Der erste Fangkorb 10 ist schirmartig ausgeführt und weist ein distales geschlossenes Ende 12 und eine proximales offenes Ende 13 auf. Beim Einführen in das Schleusenelement und insbesondere in den Katheter wird der aus einem Geflecht bestehende Fangkorb stark komprimiert, wohingegen er nach dem Herausschieben aus dem Schleusenelement und dem Katheter expandiert, um das zu entfernende Objekt von seinem proximalen Ende aus aufzunehmen.

Durch die Verstellelemente 11 kann der erste Fangkorb so positioniert werden, dass er optimal über dem Objekt ausgerichtet ist und dieses in Richtung zu dem zweiten Fangkorb schiebt, nachdem er es in sich aufgenommen hat. In Figur 1 ist die Position so dargestellt, dass der erste Fangkorb noch vor der Aufnahme des Objektes steht.

Der zweite Fangkorb 20 ist in Figur 1 als schlauchförmiges längliches Element dargestellt. Er kann jedoch auch beliebige andere Formen aufweisen, insbesondere die in Figur 2 dargestellte. Bei dieser ist das proximale Ende 23 in einem Hülsenelement 24 und einem rohrförmigen Element 25 festgelegt. Das distale Ende 22 dies zweiten Fangkorbes ist ebenso wie bei der Ausführungsform gemäß Figur 1 geöffnet. Es entsteht dadurch in der Ausführungsform gemäß Figur 2 eine Tulpenform des zweiten Fangkorbes. Insbesondere durch Vorsehen von Verstellelementen kann dadurch eine noch bessere Öffnung des distalen Endes des zweiten Fangkorbes zur Aufnahme des ersten Fangkorbes sowie des zu bergenden Objektes erzeugt werden.

Auch der zweite Fangkorb ist aus einem Gewebe hergestellt. Dieses kann zum besseren Aufweiten zumindest des distalen Endes 22 des Fangkorbes 20 aus Fäden unterschiedlichen Durchmessers gefertigt sein. Beispielsweise weist ein erster Faden 26 einen Durchmesser von 0,20 mm und ein zweiter Faden 27 einen Durchmesser von 0,15 mm auf. Es können aber auch beliebige andere Durchmesser gewählt werden. Diese Fäden unterschiedlichen Durchmessers sind miteinander verflochten, insbesondere abwechselnd verflochten. Es kann lediglich in einem Teilbereich x, der in Figur 2 angedeutet ist, ein dünnerer Faden verwendet werden und in einem zweiten Teilbereich ein dickerer Faden, so dass der erste Bereich besser aufgeweitet werden kann als der zweite Bereich. Hierdurch wird ebenfalls die Aufnahme von Objekten im distalen Bereich des zweiten Fangkorbes erleichtert. Ebenso ist die Verwendung unterschiedlicher Fadendurchmesserkombinationen in verschiedenen Bereichen möglich.

Figur 3 zeigt verschiedene Möglichkeiten, wie der, jeweilige Abschluss der Fangkörbe am distalen und/oder proximalen Ende gebildet werden kann. In Figur 3 sind lediglich drei unterschiedliche Möglichkeiten dargestellt, es sind jedoch noch zahlreiche weitere möglich. Die Möglichkeit a) gibt ein Verschlingen jeweils nebeneinander liegender Fäden an, wie dies beim Korbflechten üblich ist. Die Variante b) sieht ein Verschlingen mit kleinerem Randüberstand vor, wie dies ebenfalls vom Korbflechten bekannt ist. In der Variante c) werden Ösen gebildet, was insbesondere für das Anbringen von Verstellelementen am ersten und zweite Fangkorb vorteilhaft ist. Es kann auch eine beliebige Kombination aus den drei Varianten oder weiteren gebildet werden. Es sind ebenfalls die unterschiedlichen Fadenstärken in Figur 3 angedeutet.

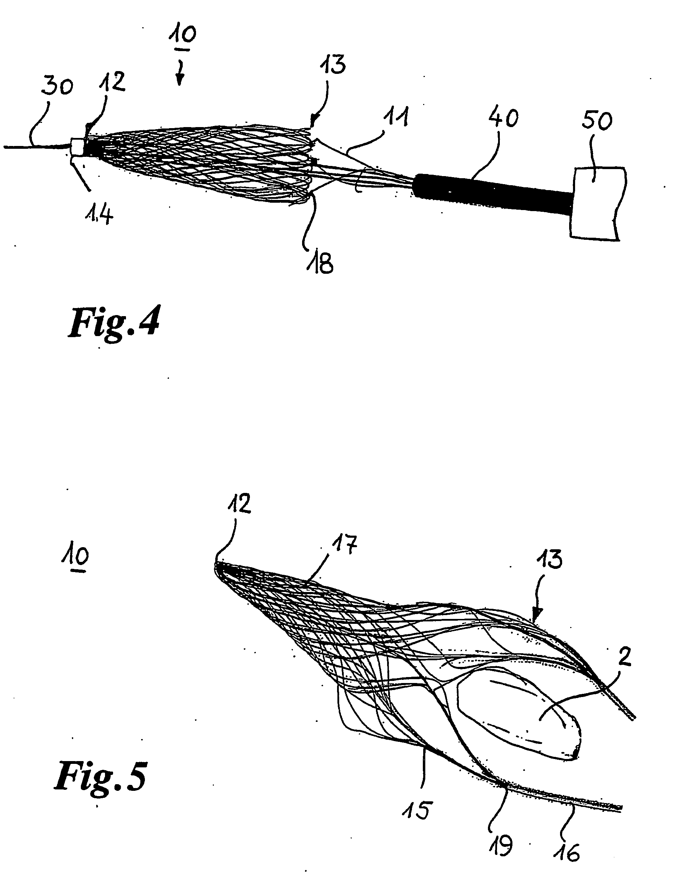

In Figur 4 ist das Detail des ersten Fangkorbes 10 dargestellt. Der erste Fangkorb ist in dieser Ausführungsform an einer Führungskanüle 14 mit seinem distalen Ende 12 befestigt. Durch die Führungskanüle ist der Führungsdraht 30 geführt. Außerhalb der Führungskanüle verlaufen die Verstellelemente 11 des ersten Fangkorbes. Führungskanüle, Verstellelemente und Führungsdraht sind durch den Katheter 40 hindurchgeführt. Dieser wiederum ist innerhalb des Schleusenelementes 50 angeordnet. Der zweite Fangkorb ist bei dieser Ausführungsform nicht vorgesehen. Es kann ein Einfangen des Objektes auch ohne den zweiten Fangkorb erfolgen.

Im Gegensatz, zu den Ausführungsformen des ersten Fangkorbes gemäß Figur 1 und Figur 4 sind bei der Ausführungsform des ersten Fangkorbes gemäß Figur 5 die Verstellelemente 11 nicht separat an dem Fangkorb befestigt, sondern mit diesem einteilig. Teile des Geflechtes des ersten Fangkorbes sind somit am proximalen Ende herausgeführt und verzweigt, wobei jeweils mehrere Fäden bzw. Drähte des Geflechtes verdrillt sind. Bei dem Verdrillen entstehen mehrere Verstellelementteile 15. Diese wiederum sind so zusammengeführt, dass lediglich zwei Verstellelementteile 16 zu dem katheter geführt werden. Zwischen den Verstellelementteilen 15, eingefangen in dem ersten Fangkorb 10 befindet sich das Objekt 2.

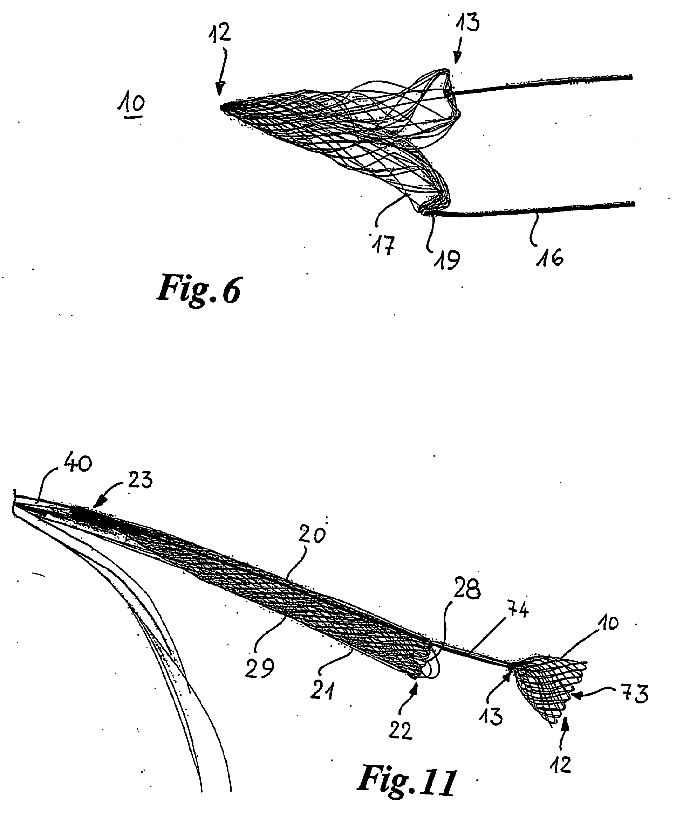

In der Ausführungsform gemäß Figur 6 sind die Verstellelementteile 15 weggelassen und die einzelnen Enden des Geflechtes des ersten Fangkorbes direkt zu zwei Verstellelementteilen 16 zusammengeführt.

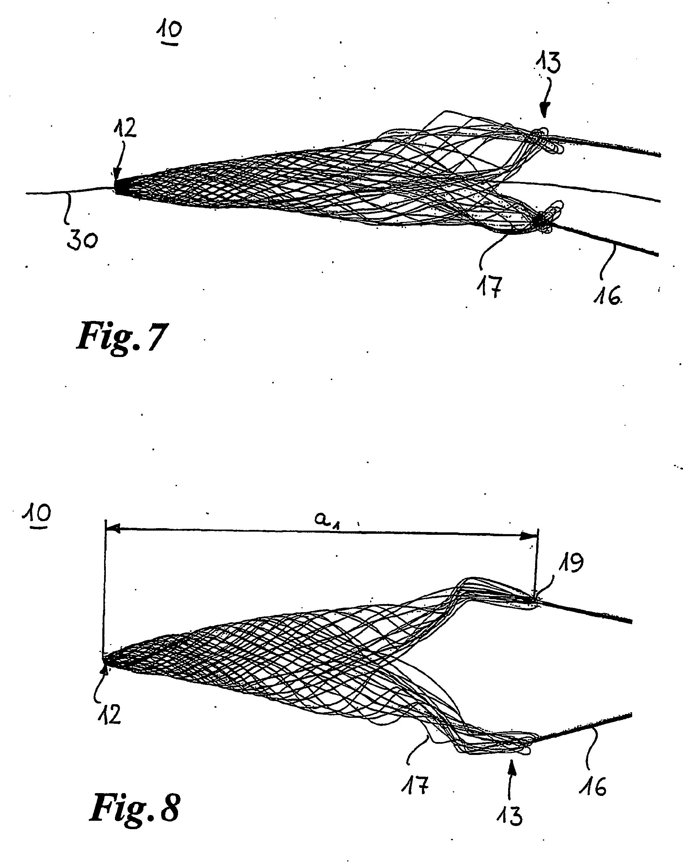

Figur 7 zeigt eine ähnliche Ausführungsform wie Figur 6, wobei in Figur 7 ein Teil der Fäden 17 am proximalen Ende Schlaufen bilden und zum distalen Ende zurückgeführt sind und nur einige Fäden den Verstellelementteil 16 bilden.

Bei der Ausführungsform gemäß Figur 8,sind die Fäden im Unterschied zu den übrigen Ausführungsformen der vorstehend beschriebenen ersten Fangkörbe 10 in einem vom proximalen Ende des Fangkorbes entfernten Bereich in zwei Teile geteilt, die dann zu den beiden Verstellelementteilen 16 zusammengeführt werden.

Bei jeder Ausführungsform der ersten Fangkörbe 10 bleibt beim Hineinziehen derselben in den Katheter und/oder das Schleusenelement der Abstand a₁ des distalen Endes 12 von der proximalen Befestigungsstelle 18 oder Austrittsstelle 19 im Wesentlichen gleich, um ein Einführen in das Schleusenelement bzw. den Katheter problemlos zu ermöglichen. Als Befestigungsstelle 18 wird hierbei die Stelle der Befestigung der Verstellelemente am ersten Fangkorb und als Austrittsstelle die Stelle des Austretens der mit dem Fangkorb einteiligen Verstellelemente aus dessen Geflecht bezeichnet.

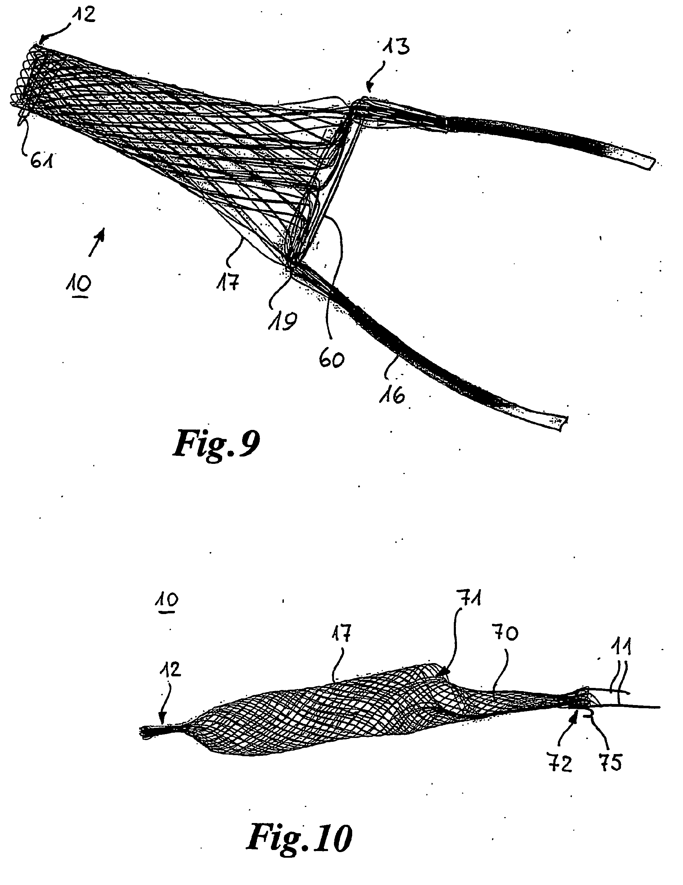

In Figur 9 ist eine weitere Ausführungsform eines erfindungsgemäß ausgeführten ersten Fangkorbs 10 dargestellt. Bei diesem sind am proximalen Ende die Fäden 17 im Wesentlichen so miteinander verflochten, dass ein gerader Abschluss entsteht. Die Enden der Fäden sind in die beiden Verstellelementteile 16 im Bereich der Austrittsstellen 19 aus dem Geflecht zusammengeführt. In diesem Bereich ist außerdem ein Reduzierelement 60 in Form einer Schlinge vorgesehen. Die Schlinge ist um den gesamten Umfang des distalen Endes des Fangkorbs 10 geführt. Das Schlingenelement ist vorzugsweise von außerhalb des Körpers des Patienten betätigbar. Hierdurch kann eine Reduzierung des Durchmessers der Öffnung am proximalen Ende des Fangkorbes erzeugt werden. Ein in den Fangkorb eingefangenes Objekt kann dadurch in diesem besser festgehalten werden. Auch am distalen Ende des ersten Fangkorbes ist ein Reduzierelement 61 vorgesehen, das ebenfalls in Form einer Schlinge vorliegt und zum Reduzieren des Durchmessers bzw. Abschnüren dieses Endes vorgesehen ist. In Figur 9 ist auch besonders gut das Ausbilden des distalen Endes hinsichtlich der Verschlingungen der einzelnen Fäden des Fangkorbes zu erkennen. Es kann also das distale Ende entweder durch durchgängiges Verweben bzw. Verflechten der einzelnen Fäden erzeugt werden oder durch Zusammenschnüren durch ein Reduzierelement wie dies in Figur 9 angedeutet ist. In Figur 9 sind die Verstellelementteile 16 als rohrförmige Elemente ausgebildet, die insbesondere aus Kunststoff oder Metall bestehen können.

Figur 10 zeigt eine weitere Ausführungsform eines erfindungsgemäß ausgeführten ersten Fangkorbes 10 mit zwei Verstellelementen 11, wobei der Fangkorb asymmetrisch, nämlich einseitig verlängert ausgeführt ist. Das Verlängerungsstück 70 ist aus dem gleichen Geflecht hergestellt wie der übrige Fangkorb. Vorzugsweise wird das einseitige Verlängerungsstück 70 so unter das einzufangende Objekt gezogen, dass dieses nahezu selbständig in die Öffnung 71 am proximalen Ende des Fangkorbs hineinrutscht. Zum Dirigieren des Verlängerungsstücks 70 können die beiden an dessen proximalem Ende 72 befestigten bzw. herausgeführten Verstellelemente 11 verwendet werden. Anstelle der beiden Verstellelemente kann auch lediglich eines verwendet werden. Am proximalen Ende 72 ist außerdem ein hakenförmiges Element 75 vorgesehen, das für den Abwurf des Fangkorbs und zum Entfernen des Fangkorbs aus dem Körper des Patienten vorteilhaft zum Angreifen genutzt werden kann. Diese Ausführungsform eines Fangkorbs eignet sich besonders als Vena-Cava-Filter.

Figur 11 zeigt eine weitere Ausführungsform einer erfindungsgemäß ausgerüsteten Extraktionsvorrichtung 1, bei der der Fangkorb 10 mit seiner Öffnung 73 von der Öffnung 28 des zweiten Fangkorbs 20 weg gerichtet ist. Bei dieser Ausführungsform ist der erste Fangkorb wiederum auf einer Führungskanüle 74 befestigt, jedoch im Unterschied zu der Figur 1 mit seinem proximalen Ende 13. Das distale Ende 12 ist in der Darstellung gemäß Figur 11 weit geöffnet, so dass darin ein einzufangendes Objekt aufgenommen werden kann.

Die Führungskanüle 74 ist durch den zweiten Fangkorb hindurchgeführt in den am proximalen Ende des zweiten Fangkorbs vorgesehenen Katheter 40 hinein. Mit seinem proximalen Ende 23 ist der zweite Fangkorb an dem Katheter 40 befestigt, um ihn vermittels der Verstellelemente 21 in seiner Form und Position verändern zu können, das heißt expandieren und komprimieren zu können. Die Verstellelemente 21 sind in die Mantelfläche 29 des zweiten Fangkorbs zum Teil eingearbeitet bzw. durch das Geflecht der Mantelfläche geflochten. Hierdurch ist eine besonders gute Betätigung des zweiten Fangkorbes möglich. Bevorzugt sind, wie in Figur 11 ebenfalls dargestellt, die Verstellelemente 21 des zweiten Fangkorbs außerhalb des Katheters zu dessen proximalen Ende geführt, um eine Betätigung von außerhalb der Operationsstelle zu ermöglichen. Ein Schleusenelement, durch das der Katheter 40, die Führungskanüle 74 sowie die Verstellelemente 21 geführt werden können, wird durch die Haut des Patienten hindurch in den entsprechenden Hohlraum, in dem das zu fangende Objekt sitzt, bis kurz vor dieses geführt. Dies ist in Figur 11 jedoch nicht zu sehen.

Der Extraktionsvorgang bzw. das Einfangen eines Objektes und Herausholen aus dem Hohlraum wird dadurch vorgenommen, dass das Schleusenelement bzw. ein Katheter gegebenenfalls mit Innenmandrin über einen Führungsdraht in dem entsprechenden Hohlraum, insbesondere einem Gefäß platziert wird. Der Katheter wird über das einzufangende Objekt, insbesondere einen Thrombus, bis in dessen Peripherie platziert. Nachfolgend wird zunächst der erste Fangkorb 10 freigesetzt. Anschließend geschieht dies ebenso mit dem zweiten Fangkorb 20, wobei dieser jedoch vor dem Thrombus aus dem Katheter herausgeschoben und entfaltet wird. Dadurch wird der Thrombus beidseitig von den Fangkörben flankiert. Im Falle der Ausführungsform gemäß Figur 11 werden jedoch beide Fangkörbe vor dem Thrombus freigesetzt, so dass dieser nicht nur vor dem zweiten, sondern auch vor dem ersten Fangkorb platziert ist. Nach dem Freisetzen der Fangkörbe wird der Thrombus mit dem ersten Fangkorb eingefangen und dieser in den zweiten Fangkorb 20 zurückgezogen, zusammen mit dem Thrombus. Zur weiteren Bergung des Thrombus werden sodann beide Fangkörbe in dem ineinandergezogenen Zustand in das Schleusenelement zurückgezogen. Hierbei findet eine Kontraktion beider Fangkörbe und dadurch ein Ausquetschen des Thrombus statt. Dies erfolgt vorteilhaft in der Pulmonalarterie oder Vena cava.

Der Abtransport eines anderen Objekts, beispielsweise eines Fremdkörpers oder durch den Organismus selbst gebildeten Körpers, wie eines Nieren- oder Gallensteins, erfolgt in entsprechender Weise, wobei der Fremdkörper, je nach Konsistenz, keinen Quetschvorgang erfährt. Er wird lediglich aus dem Hohlraum geborgen und nach außerhalb des Körpers des Patienten gezogen.

Zur Wegeinhaltung ist der Führungsdraht vorgesehen. Zum besseren Dirigieren des ersten Fangkorbes kann die Führungskanüle an diesem, wie in Figur 4 und 7 sowie Figur 11 gezeigt, verwendet werden. Wie insbesondere Figur 11.zu entnehmen, sind die Verstellelemente, vorzugsweise Drähte, an den Enden, hier dem distalen Ende 22 des zweiten Fangkorbs befestigt. Hierdurch kann sichergestellt werden, dass sich der zweite Fangkorb zum Hineinziehen des ersten Fangkorbs optimal öffnen lässt.

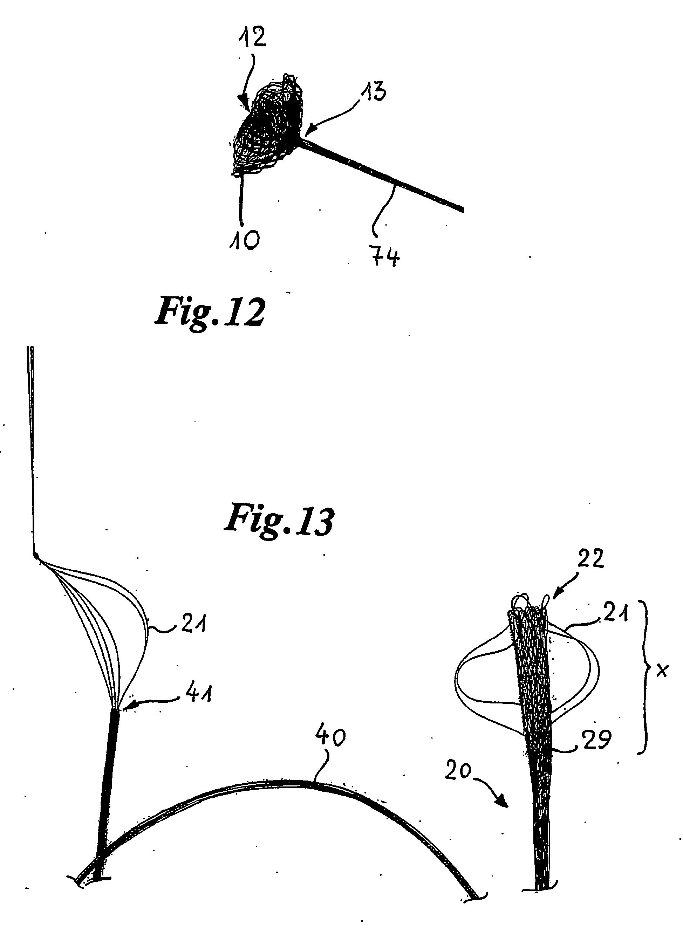

In Figur 12 ist eine weitere Ausführungsvariante für den ersten Fangkorb 10 dargestellt. Dieser ist im Unterschied zu der Ausführungsform gemäß Figur 11 um 180° umgeklappt, weist nun also wiederum mit seiner Öffnung zu der distalen Öffnung des zweiten Fangkorbs. Die Führungskanüle ist dadurch wieder am distalen Ende des ersten Fangkorbes befestigt. Mit dieser Ausführungsvariante lassen sich besonders gut Emboli einfangen, da diese häufig weiter gestreut in einem Gefäß vorliegen und mit dieser Ausführungsvariante ein sich verhältnismäßig weit öffnender Fangkorb erstellen lässt. Aus dem in Figur 11 gezeigten Fangkorb 10 kann durch Umklappen um 180° der in Figur 12 gezeigte erzeugt werden, z.B. auch durch Ziehen an Verstellelementen, die am offenen Ende des Fangkorbs befestigt werden können.

In Figur 13 ist der zweite Fangkorb 20 mit seinen Verstellelementen 21 und einem Katheter 40 dargestellt. Der zweite Fangkorb ist im Wesentlichen komprimiert gezeigt, wobei die Verstellelemente, die zur Expansion beitragen, besonders verdeutlicht sind. Da gerade der distale Bereich x zur Aufnahme des ersten Fangkorbs bzw. des zu bergenden Objektes expandiert werden soll, sind in diesem Bereich die Verstellelemente aus der Mantelfläche herausgezogen gezeigt. Hierdurch wird auch deutlich, dass sie am distalen Ende 22 des zweiten Fangkorbs befestigt bzw. an diesem Ende durchgeschlungen sind, so dass ein Verstellelement jeweils eine Hin- und Rückrichtung einnimmt. Am proximalen Ende 41 des Katheters sind die Verstellelemente herausgeführt. An dieser Stelle kann eine Betätigung derselben erfolgen, insbesondere durch einen nicht dargestellten Handgriff.

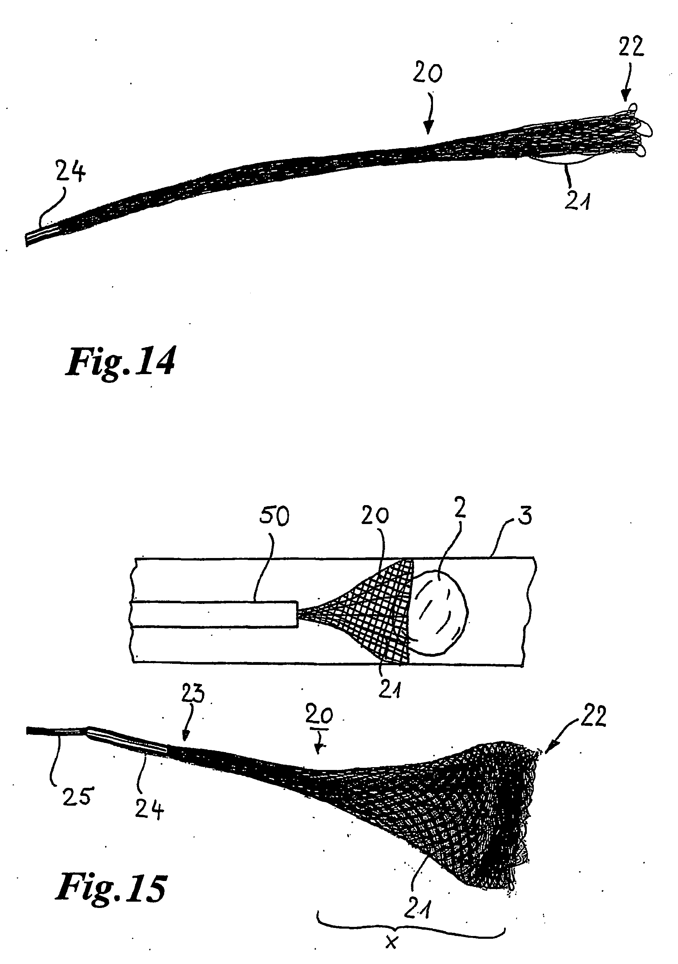

Die Figuren 14 bis 19 zeigen verschiedene Schritte des Einfangens eines Objektes, beispielsweise eines Fremdkörpers in einem Gefäß durch den zweiten Fangkorb 20. In Figur 14 ist dabei der langgestreckte nicht expandierte Fangkorb gezeigt, ähnlich wie in der Darstellung in Figur 13. Das distale Ende 22 des zweiten Fangkorbes ist dabei noch im Wesentlichen geschlossen. Die Figur 15 zeigt demgegenüber ein bereits geöffnetes distales Ende 22 des Fangkorbs 20. Die Öffnung bzw. das Expandieren des Fangkorbes in diesem distalen Bereich x erfolgt durch die Verstellelemente 21. In dem oberen Zeichnungsteil der Figur 15 ist der Fangkorb aus dem Schleusenelement 50 herausgeschoben innerhalb eines Gefäßes gezeigt, wobei er zum Teil bereits über das Objekt 2 geschoben ist.

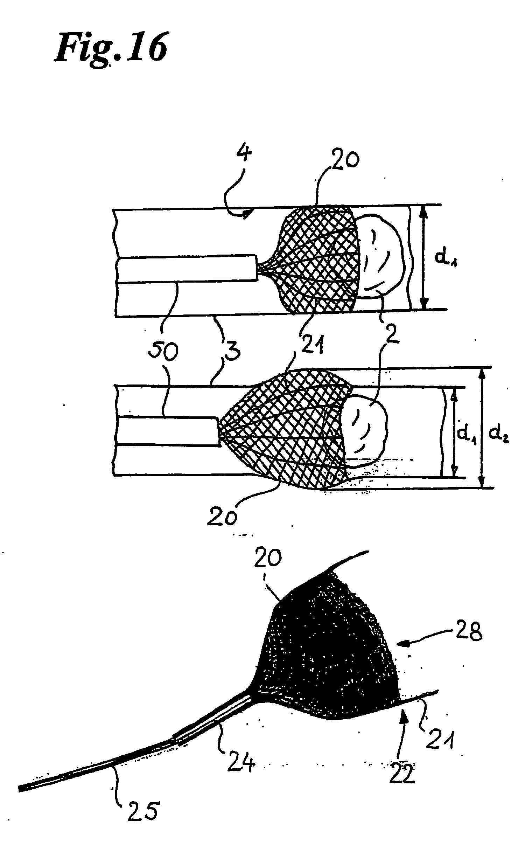

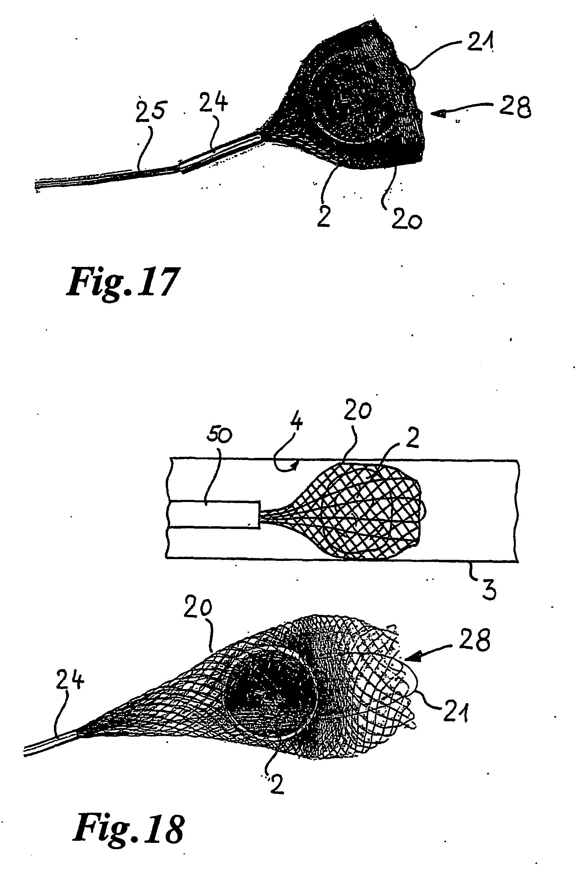

In der Figur 16 ist der weiter expandierte zweite Fangkorb 20 gezeigt. In der obersten Ansicht ist der Fangkorb gerade so weit aufgeweitet, dass er sich an die Gefäßinnenwand 4 vollständig anlegt. In der darunter dargestellten Position weitet der Fangkorb beim weiteren Expandieren die Gefäßwand partiell auf, so dass der Gefäßdurchmesser d₁ partiell einen Durchmesser von d₂ einnimmt. Durch diese Gefäßaufweitung rutscht das Objekt 2 selbständig in die Öffnung 28 des Fangkorbs hinein. Das in den zweiten Fangkorb hineingesprungene bzw. -gerutschte Objekt ist in Figur 17 dargestellt. Hierbei ist der Fangkorb noch endseitig aufgeweitet gezeigt. -

Figur 18 zeigt einen weiteren Schritt des Einfangens und Bergens des Objektes 2, wobei in diesem Schritt wieder eine Entlastung der Verstellelemente 21 erfolgt. Hierdurch zieht sich der schlauchartige Fangkorb im distalen Bereich x wieder zusammen, beginnend im Bereich der Öffnung 28. Hierdurch wird das Objekt 2 daran gehindert, wieder aus dem Fangkorb herauszurutschen. Dem oberen Teil der Figur 18 kann außerdem entnommen werden, dass beim Kontrahieren des Fangkorbs dieser wieder von der Gefäßinnenwand 4 gelöst wird.

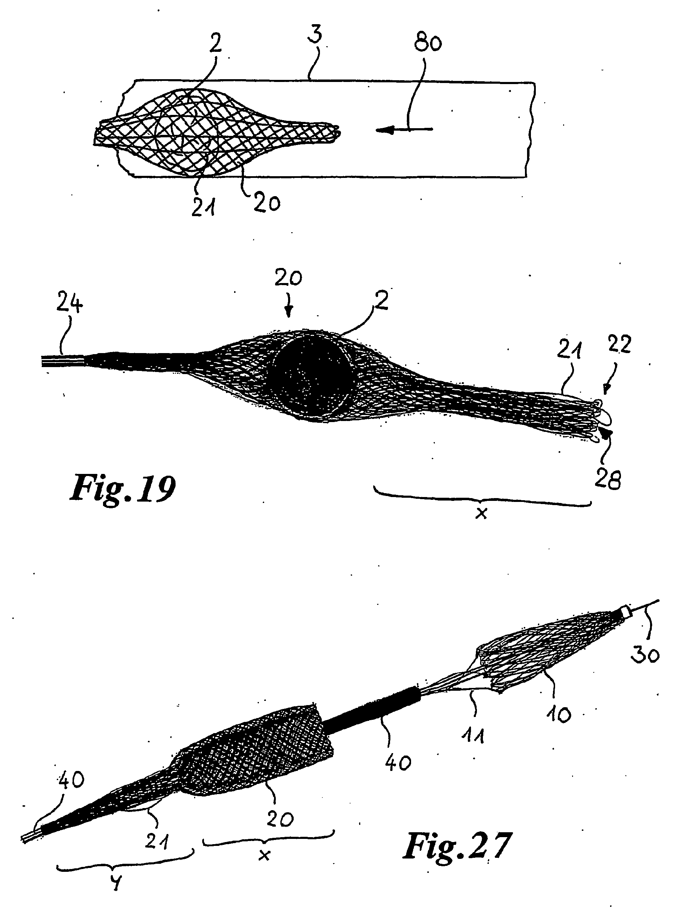

In dem letzten Schritt, der in Figur 19 dargestellt ist, sind die Verstellelemente 21 so weit gelockert, dass sich das Gewebe des zweiten Fangkorbes strumpfartig im distalen Bereich x wieder zusammenzieht, so dass die Öffnung 28 am distalen Ende 22 des Fangkorbs wieder nahezu geschlossen ist. Das Objekt 2 ist vollständig innerhalb des Fangkorbs aufgenommen. Wie aus dem oberen Teil der Figur 19 zu entnehmen, kann nun der Fangkorb wieder aus dem Gefäß bzw. Hohlraum zurückgezogen werden, was durch den Pfeil 80 angedeutet ist.

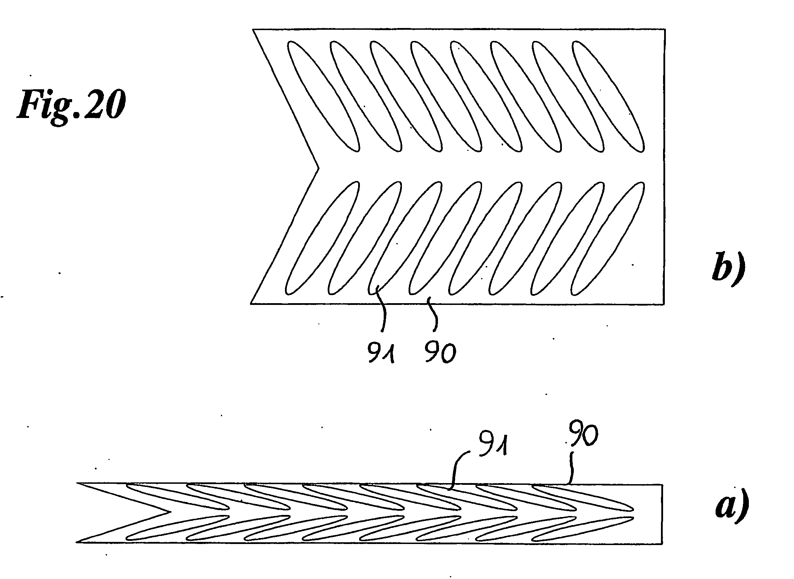

Wie insbesondere den Figuren 15, 16 und 17 zu entnehmen ist, wird beim Aufweiten des Fangkorbs auch dessen Verkürzung bewirkt. Je mehr sich hier der Fangkorb verkürzt, desto effektiver wird seine Aufweitung und desto besser kann ein Objekt eingefangen werden. Dieser Effekt kann nicht nur mit einem Drahtgeflecht oder allgemein Geflecht des Fangkorbs erzeugt werden sondern auch durch Vorsehen eines geschlitzten Rohres 90 wie in Figur 20 zu sehen. Der obere Teil der Figur 20 zeigt dabei das komprimierte geschlitzte Rohr mit Schnitten 91, wohingegen der untere Teil der Figur 20 das expandierte mit Schnitten 91 versehene Rohr zeigt. Die Anordnung der Schnitte 91 ist in der dargestellten Ausführungsform ähnlich wie bei Rippen eines Skelettes vorgesehen, wobei die einzelnen Schnitte im Wesentlichen parallel zueinander angeordnet sind. Es kann jedoch auch jede beliebige andere Form von Schnitten gewählt werden, mit der ein Expandieren und nachfolgendes Komprimieren des Rohres zum Verändern von dessen Querschnitt möglich ist. Durch Vorsehen besonders langer Schnitte ist eine große Querschnittsveränderung möglich. Die in Figur 20 dargestellte Ausführungsvariante eines geschlitzten Rohres eignet sich besonders für den zweiten Fangkorb. Für den ersten Fangkorb kann eine Variante, wie sie in der DE 100 00 137 offenbart ist, verwendet werden, insbesondere die Variante, die dort in der Figur 1 dargestellt ist.

Die vorliegende Extraktionsvorrichtung kann auch im Zusammenhang mit einem Endoskop verwendet werden. Bei der Endoskopie sind Lichtquellen, eine optische Einrichtung sowie ein Arbeitskanal angeordnet. Zur Verwendung mit einem Endoskop wird beispielsweise der zweite Fangkorb 20 an den ersten Fangkorb 10 von außen angeklemmt. Dies kann durch die Selbstklemmkraft aufgrund der Vorspannung des Gewebes oder geschlitzten Rohres des zweiten Fangkorbs erfolgen. Diese Selbstklemmkraft wird unabhängig vom Einsatz in Verbindung mit einem Endoskop ansonsten insbesondere zum Halten des eingefangenen Objektes genutzt. Der erste Fangkorb 10 wird nachfolgend durch den Arbeitskanal eingebracht. Der zweite Fangkorb 20 läuft von außen aufgrund des Anklemmens an dem ersten Fangkorb nach. Während des Transportes zu der Operationsstelle hält sich der zweite von außen aufgeklemmte Fangkorb selbst fest. An der Operationsstelle werden dann beide Fangkörbe geöffnet. Gegebenenfalls kann ein Schleusenelement über beide Fangkörbe geschoben mit in den Arbeitskanal eingebracht werden. Besonders eignen sich bei dieser Verwendungsform Endoskope mit einem Durchmesser von 3 mm. Je nach Anwendungsfall können jedoch auch größere Endoskope verwendet werden, beispielsweise Endoskope mit einem Durchmesser von 10-15 mm. Die Funktion des Einfangens des zu bergenden Objektes entspricht der bereits vorstehend beschriebenen.

Die Figur 27 zeigt eine Kombination aus verschiedenen der vorstehend beschriebenen Ausführungsformen von erstem und zweitem Fangkorb. Der zweite Fangkorb 20 ist im distalen Bereich x selbständig öffnend. Dieser Teil passt sich und legt sich dabei an die Gefäß- oder Hohlraumwand an. Der proximale Teilbereich y bleibt dabei komprimiert oder gefaltet, kann jedoch aktiv durch Ziehen an den Verstellelementen 21 geöffnet werden. Nach dem Entlasten der Verstellelemente komprimiert sich dieser proximale Teilbereich wieder und hält dadurch aufgrund seiner eingeprägten Form und Formhaltekraft einen Thrombus, Emboli, etc. fest.

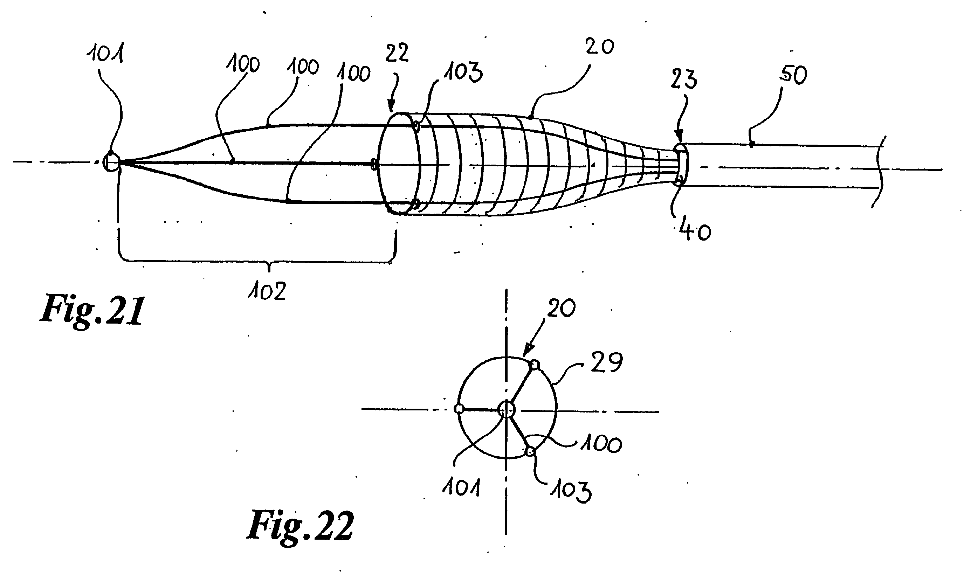

In den Figuren 21 und 22 ist eine weitere Ausführungsvariante des Fangkorbes 20 dargestellt. Diese ähnelt der in Figur 2 dargestellten. Die in dieser Ausführungsvariante verwendeten Verstellelemente 100 sind über das distale Ende 22 des Fangkorbs 20 hinausgeführt. Mit Abstand zu dem distalen Ende des Fangkorbs 20 sind sie zusammengeführt und mit einer atraumatischen Kugel 101 versehen. Der zwischen dem distalen Ende des zweiten Fangkorbes 20 und der atraumatischen Kugel 101 gebildete Abschnitt der Verstellelemente 100 dient ebenfalls als Fangkorb und ist daher mit dem Bezugszeichen 102 bezeichnet. Der Fangkorb 102 ist ohne Ummantelung offen ausgeführt. Die Verstellelemente sind in Ösen 103 am distalen Ende des zweiten Fangkorbs 20 geführt. Am proximalen Ende des Fangkorbes 20 ist dieser im Katheter 40 festgelegt. Es kann auch eine weitere Ummantelung durch das Schleusenelement 50 erfolgen, wie dies in den vorstehenden Ausführungsformen dargestellt ist.

Der Fangkorb 20 kann wiederum aus einem Metallnetz oder Textilnetz bestehen, wobei sowohl die Verstellelemente 100 als auch der Fangkorb 20 aus einem elastischen selbst expandierenden Material bestehen. Hier kann beispielsweise ein Metalldraht verwendet werden, insbesondere Nitinol als Formgedächtnismaterial.

Durch Vorsehen der Ösen 103 können die Verstellelemente gegenüber dem Fangkorb 20 verkürzt werden, um ein eingefangenes Objekt, insbesondere embolisches Material, in den Fangkorb 20 einzuziehen. Durch Vorschub des Schleusenelementes 50 werden der geschlossene Fangkorb 20 und der offene Fangkorb 102 radial komprimiert und nachfolgend vollständig von dem Schleusenelement ummantelt. Umgekehrt expandieren beide Fangkörbe beim Zurückziehen des Katheters aufgrund ihrer eingeprägten Elastizität und Form auf ihren voreingeprägten maximalen Entfaltungsdurchmesser. Dies entspricht den in den vorstehenden Figuren gezeigten Ausführungsformen von Fangkörben. Zum Einfangen embolischen Materials bzw. allgemein eines Objektes ist es nicht unbedingt erforderlich, den offenen Fangkorb 102 in den geschlossene Fangkorb 20 hineinzuziehen. Es kann vielmehr auch der geschlossene Fangkorb 20 gegenüber dem offenen Fangkorb 102 verlängert bzw. vorgeschoben werden, so dass letztlich das in dem offenen Fangkorb eingefangene Objekt wiederum in den geschlossenen Fangkorb 20 gelangt.

Es können vergleichsweise wenige Verstellelemente 100 vorgesehen werden, insbesondere lediglich drei oder vier, wobei diese als Führungsdrähte für den Vorschub des Fangkorbes 20 im entfalteten Zustand dienen. Außerdem behindern wenige Verstellelemente das Einfangen eines Objektes kaum.

Die Frontansicht in Figur 22 zeigt, dass alle drei Verstellelemente in einem gleichmäßigen Abstand zueinander, nämlich einem Winkel von etwa 120°, auf den Umfang der Mantelfläche des Fangkorbs 20 verteilt angeordnet sind. Es kann jedoch auch eine ungleichmäßige Verteilung über diesen Mantelumfang erfolgen.

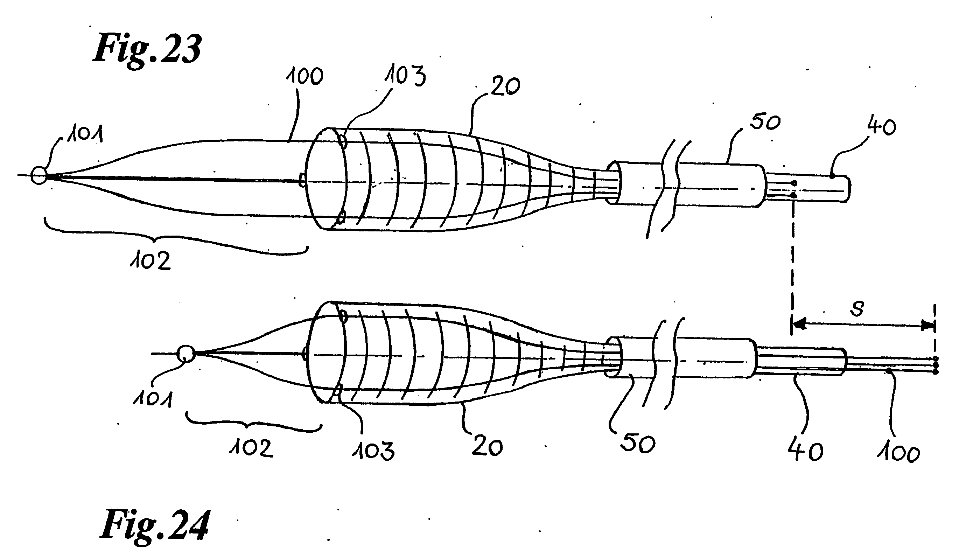

Die Figuren 23 und 24 zeigen die Relativverstellung des offenen Fangkorbes 102 gegenüber dem geschlossenen Fangkorb 20. In dieser Darstellung ist außerdem der Katheter 40 innerhalb des Schleusenelementes 50 dargestellt, mit welchem Katheter der Fangkorb 20 an seinem proximalen Ende fest verbunden ist. Anstelle von Ösen 103 können auch andere Führungselemente verwendet werden. Diese dienen insbesondere zur Stabilisierung der koaxialen Verschiebung des Fangkorbes 102 gegenüber dem Fangkorb 20 bzw. umgekehrt. Es wird hierdurch eine rein axiale Bewegung zugelassen, wohingegen eine radiale oder eine zum Umfang tangentiale Bewegung im Wesentlichen verhindert wird.

Die Strecke s, um die die Verstellelemente 100 am proximalen Ende des Katheters herausgezogen werden, entspricht im Wesentlichen der Strecke, um die der offene Fangkorb 102 in den geschlossenen Fangkorb 20 hineingezogen wird und sich somit der offene Fangkorb verkürzt.

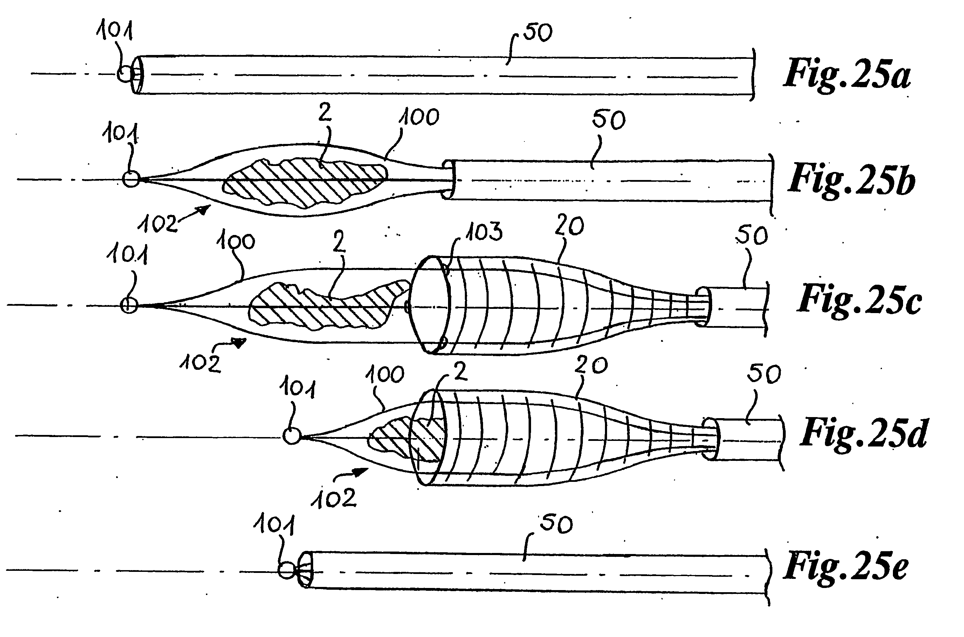

Figur 25 zeigt eine Abfolge des Einfangvorganges eines Objektes 2 mit der in den Figuren 21 bis 24 dargestellten Ausführungsform einer erfindungsgemäßen Extraktionsvorrichtung. Hierbei wird zunächst das Schleusenelement 50 mit einem Innenmandrin über einen Führungsdraht in beispielsweise eine pulmonalarterielle Strombahn eingebracht. Nach Entfernen des Innenmandrins und gegebenenfalls des Führungsdrahtes wird zunächst der offene Fangkorb 102 aus dem Schleusenelement vorgeschoben, bis die atraumatische Kugel 101 aus dem distalen Ende des Schleusenelementes hervortritt (s. Figur 25 a)). Nach dem Zurückziehen des Schleusenelementes wird der offene Fangkorb 102 freigegeben. Die Verstellelemente 100 expandieren und können um das einzufangende Objekt herum positioniert werden, wie dies in Figur 25 b) dargestellt ist. Während des Einfangvorganges kann die gesamte Extraktionsvorrichtung rotieren und dadurch das Einfangen unterstützt werden. Beim weiteren Zurückziehen des Schleusenelementes 50 wird auch der Fangkorb 20 freigegeben, wie dies in Figur 25 c) dargestellt ist. Anschließend wird der offene Fangkorb 102 in den geschlossenen Fangkorb 20 zurückgezogen und dabei verkürzt, wobei das eingefangene Objekt in den Fangkorb 20 hineingezogen wird, wie dies in Figur 25 d) dargestellt ist. In dem fünften in Figur 25 dargestellten Schritt (Figur 25 e)) wird das Schleusenelement gegenüber dem Fangkorb 20 nach distal geschoben und dadurch beide Fangkörbe vollständig in den Katheter eingezogen und darin komprimiert. Während dieses Vorgangs kann der flüssige Anteil des eingefangenen Objektes, insbesondere eines Thrombus oder Embolus, durch die Maschen der Ummantelung des geschlossenen Fangkorbs 20 abgepresst werden. Im Inneren des Fangkorbes verbleibt dann die zelluläre und gewebige Matrix des Thrombus oder Embolus. Diese kann durch Zurückziehen lediglich des Katheters oder Schleusenelementes nach außerhalb des Körpers des Patienten extrahiert werden. Eine Wiederholung des Vorgangs ist nach Säubern der beiden Fangkörbe und Wiedereinführen der beiden über das noch in der Pulmonalarterie liegende Schleusenelement möglich, bis die Pulmonalarterie rekanalisiert ist.

In einer alternativen Ausführungsform ist es auch möglich, dass die Verstellelemente 100 innerhalb der Mantelfläche 29 des Fangkorbes 20 verlaufen. Hierdurch kann ein besonders großer Fangkorb 20 verwendet werden, der sich vollständig an einer Gefäßinnenwand anlegen kann, ohne dass außen geführte Verstellelemente 100 oder Ösen diese verletzten. Auch ein Verflechten der Verstellelemente mit der Mantelfläche 29 ist möglich.

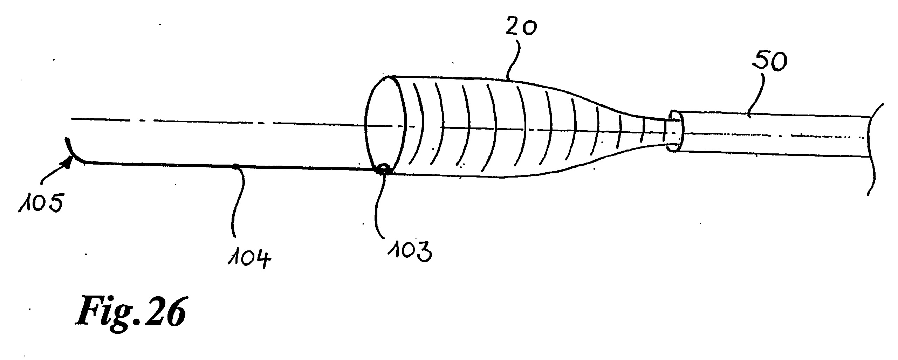

In einer weiteren Ausführungsform, die in Figur 26 dargestellt ist, ist anstelle dreier Verstellelemente 100 lediglich ein Verstellelement 104 vorgesehen. Dieses ist wiederum durch eine Öse 103 geführt, liegt jedoch auf der Innenseite des Fangkorbes 20 an. Auch das Verstellelement 104 ist axial verschiebbar und übernimmt die Funktion eines Führungsdrahtes. Das Verstellelement ist an seinem distalen Ende 105 gekrümmt, um sich an einzufangenden Objekten festhalten zu können. Hierzu kann das distale Ende 105 beispielsweise auch löffelartig ausgebildet sein, um das Festhalten zu erleichtern.

Die Verstellelemente und insbesondere auch das eine Verstellelement 104 setzen sich entlang der Mantelfläche des Fangkorbs 20 in der Katheter- bzw. Schleusenelementachse fort, wobei sie gleichzeitig eine relative axiale Bewegung zulassen.

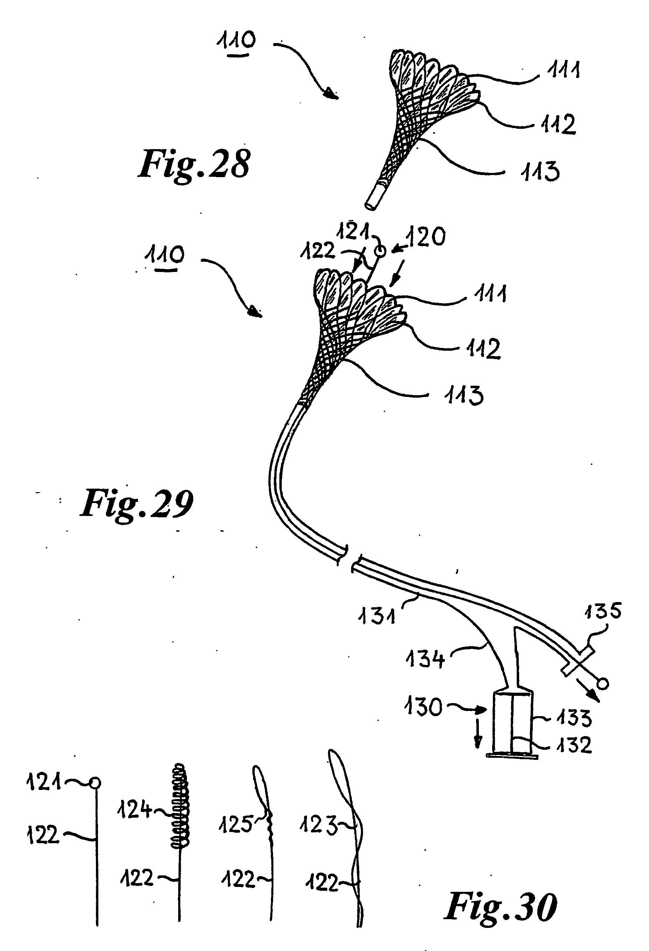

Fig. 28 zeigt eine weitere Ausführungsform eines erfindungsgemäßen Fangkorbs 110. Im Unterschied zu beispielsweise den Fangkörben, die in den Fig. 17 und 18 dargestellt sind, weist der Fangkorb 110 eine Beschichtung 111 auf. Diese ist in den Zwischenräumen 112 der netzartigen Struktur 113 des Fangkorbs vorgesehen. Beispielsweise wird sie durch Tauchen der netzartigen Struktur des Fangkorbs aufgebracht. Diese Beschichtung ist als eine membranartige Struktur bzw. ein Film innerhalb der netzartigen Struktur des Fangkorbs ausgebildet. Zum Ausbilden der membranartigen Struktur eignen sich besonders filmbildende Materialien, wie beispielsweise ein aus einem oder mehreren Monomeren gebildetes natürliches oder synthetisches Polymer, insbesondere gebildet durch Polyaddition, Polymerisation oder Polykondensation, insbesondere ein Polycarbonat, Polyester, Polyamid, Polyolefin oder Polyurethan. Auch Polystyrole eignen sich für die Beschichtung. Je nach Anwendungsfall kann ein Material gewählt werden, das eine größere oder geringere Flexibilität bzw. Oberflächenspannung aufweist. In jedem Falle sollte es so flexibel sein, dass ein Einfangvorgang nicht durch die Beschichtung behindert wird, sondern vielmehr möglichst durch diese noch verbessert werden kann. Gerade für das Einfangen sehr kleiner Objekte oder Objektteile eignet sich das Vorsehen einer solchen Beschichtung, um diese beim Bergen nicht aus dem Fangkorb zu verlieren. Eine Beschichtung kann bei jeder der vorstehend beschriebenen Ausführungsformen von Fangkörben vorgesehen werden.

Alle vorstehend beschriebenen Ausführungsformen von Fangkörben können eine Beschichtung der einzelnen Elemente ihres Strukturaufbaus aufweisen, insbesondere der Drähte, aus denen sie geformt sind. Für eine solche Beschichtung eignet sich eine bioaktive Oberflächenbeschichtung, eine Beschichtung mit Heparin, eine Karbonisierung von Nitinol bei Verwendung von Nitinol als Formgedächtnismaterial für die Ausbildung des zumindest einen Fangkorbs, nanotechnische oder biotechnologische Beschichtungen, das Aufbringen von röntgendichten Partikeln, insbesondere Tantal-Partikeln, eine einen Wirkstoff freisetzende Beschichtung, z.B. eine Beschichtung mit Polymeren, insbesondere Polyurethan, die mit Wirkstoffen, z.B. Medikamenten, gesättigt sind/ist. Als besonders vorteilhaft erweist sich eine solche Anreicherung mit Medikamenten und anderen Wirkstoffen bei der Verwendung der Extraktionsvorrichtung als Vena-Cava-Filter. Die Polymere können auch mikroporös sein. Durch eine solche Beschichtung der Drähte bzw. Elemente der Struktur des Fangkorbs kann die Bildung von (neuen) Thromben ebenso wie das Anwachsen von Endothelzellen verhindert werden. Das Material der Beschichtung wird bevorzugt so ausgewählt, dass es im Körper des Patienten keine Entzündungen hervorruft und nicht zur Bildung von Thromben führt (=Tarnkappenbeschichtung).

Sofern der eingefangene Thrombus bzw. das eingefangene Material zu große Abmessungen aufweist, um in einen Katheter hineingezogen werden zu können, wird vorteilhaft eine Einrichtung 120 zum Zerschneiden des Thrombus im Bereich des Fangkorbs vorgesehen (siehe Fig. 29). Eine solche Einrichtung 120 kann beispielsweise ein mit einer Kugel 121 oder einer ähnlichen Einrichtung versehener Draht 122 sein, der innerhalb des Fangkorbs, insbesondere des Fangkorbs gemäß Fig. 28, der durch Vorsehen der Beschichtung nach außen abgeschlossen ist, angeordnet und in diesen zurückgezogen werden kann und dabei den Thrombus bzw. das eingefangene Objekt zerschneidet. Anstelle eines mit einer Kugel versehenen Drahtes kann auch eine beliebige andere Einrichtung zum Zerschneiden des Thrombus bzw. eingefangenen Objekts vorgesehen werden, beispielsweise auch eine zusätzliche Drahtschlinge 123, ein wendelförmiger Abschnitt 124, ein schlingenförmiger Abschnitt 125, eine Kombination verschiedener Verdickungen oder dergleichen. In Fig. 30 sind vier verschiedene Ausführungsvarianten dargestellt.

Zum Einführen des Fangkorbs mit einem mit einer Materialverdickung versehenen Draht wird zunächst ein Führungsdraht durch einen Katheter bis zu der Stelle im Körper des Patienten vorgeschoben, an der das Objekt, insbesondere der Thrombus, entfernt werden soll. Nachfolgend wird über den Führungsdraht ein Schleusenelement vorgeschoben und anschließend der Führungsdraht wieder entfernt. Die Materialverdickung insbesondere in Form einer Kugel wird auf einem neu einzuführenden Draht oder einem entsprechenden Element durch das Schleusenelement hindurch zum Objekt vorgeschoben und dieses dort zerteilt. Ist der distale Abschnitt eines Drahtes oder ähnlichen elementes mit einem wendel- oder schlingenförmigen Abschnitt versehen, kann dieser Draht bzw. dieses Element selbst als Führungsdraht verwende werden. Hierdurch entfällt das vorherige Einführen eines Führungsdrahtes.

Anstelle der Verwendung eines Drahtes kann ebenso ein Ballonkatheter zusammen mit einem Stent oder dergleichen expandierbar rohrförmigen Element verwendet werden. Eine solche Ausführungsform eignet sich besonders für die Verwendung bei z.B. Arteriosklerose, um das an der Gefäßwandung abgelagerte Calcium-Material als Fremdköroper zu entfernen. Beispielsweise wird der Ballonkatheter zusammen mit dem Fangkorb gemäß Fig. 28 in das Gefäß des Patienten eingeschoben, z.B. die Arteria carotis, der Fangkorb geöffnet, der Fremdkörper eingefangen und entfernt. Durch den Ballonkatheter kann eine Aufweitung des Gefäßes und damit ein Freisetzen von an der Wandung anhaftenden Fremdkörpern erreicht werden.

Um das Rücktransportieren der eingefangenen bzw. zerteilten Thrombusteile und Objekte zu erleichtern, kann vorteilhaft eine Einrichtung 130 zum Absaugen der Thrombenteile bzw. Objektteile und Objekte vorgesehen werden. Eine solche Absaugeinrichtung umfasst beispielsweise eine Kanüle 131, die im Bereich des Fangkorbs endet und über die die Thrombenteile bzw. Objektteile und Objekte abgesogen werden können. Hierzu wird am anderen Ende der Kanüle ein Unterdruck erzeugt, beispielsweise über einen Kolben 132 in einem Zylinderteil 133. Die Kanüle kann vorteilhaft durch einen Katheter zu der Stelle im Körper des Menschen oder Tieres gebracht werden, an der der Thrombus bzw. das Objekt entfernt werden soll. Eine solche Absaugeinrichtung kann sowohl bei den Ausführungsformen mit normaler netzartiger Struktur als auch bei der beschichteten Ausführungsform gemäß Fig. 28, wie in Fig. 29 gezeigt, vorteilhaft eingesetzt werden, vor allem auch bei Vorsehen einer Einrichtung zum Zerschneiden bzw. Zerteilen des Objekts bzw. der Objektteile. Die Kanüle 131 weist proximal einen Abzweigstutzen 134 auf, der das Zylinderteil 133 mit Kolben 132 und ein Griffteil 135 zum Angreifen beim Betätigen des Drahtes 122 aufweist. Die Pfeile in Fig. 29 geben die Rückzugsrichtung und die Richtung zum Erzeugen eines Unterdrucks zum Absaugen von Objekten an.

Neben den im Vorstehenden genannten und beschriebenen Ausführungsformen sind noch zahlreiche weitere möglich, insbesondere auch Kombinationen der beschriebenen Formen, bei denen jeweils bei Vorsehen eines ersten und eines zweiten komprimierbaren und expandierbaren Fangkorbes, zwischen denen Objekte eingefangen werden können, wobei die Fangkörbe ineinander gezogen werden können, zumindest der eine Fangkorb im expandierten Zustand schirmartig ausgebildet ist. Bei Vorsehen zumindest eines komprimierbaren und expandierbaren Fangkorbes mit einem distalen und einem proximalen Ende kann zumindest ein Verstellelement an dem distalen und/oder proximalen Ende so befestigt werden, dass der zumindest eine Fangkorb durch diese dirigiert und in seiner Form verändert werden kann. Zusätzlich oder alternativ ist das Vorsehen einer Beschichtung des Fangkorbs möglich. Insbesondere können auch Kombinationen aus geschnittenen Rohren und Geflechten, Gelegen oder Geweben bei entsprechend geeigneter Materialwahl für den oder die Fangkörbe verwendet werden.

### Bezugszeichenliste

- 1: Extraktionsvorrichtung
- 2: Objekt
- 3: Hohlraum
- 4: Gefäßinnenwand
- 10: erster Fangkorb
- 11: Verstellelement
- 12: distales Ende
- 13: proximales Ende
- 14: Führungskanüle
- 15: Verstellelementteil
- 16: Verstellelementteil
- 17: Faden
- 18: Befestigungsstelle
- 19: Austrittsstelle
- 20: zweiter Fangkorb
- 21: Verstellelement
- 22: distales Ende
- 23: proximales Ende
- 24: Hülsenelement
- 25: rohrförmiges Element
- 26: erster Faden
- 27: zweiter Faden
- 28: Öffnung
- 29: Mantelfläche
- 30: Führungsdraht
- 40: Katheter
- 41: proximales Ende
- 50: Schleusenelement
- 60: Reduzierelement
- 61: Reduzierelement
- 70: Verlängerungsstück
- 71: Öffnung
- 72: proximales Ende
- 73: Öffnung
- 74: Führungskanüle
- 75: Hakenförmiges Element
- 80: Pfeil
- 90: geschlitztes Rohr
- 91: Schnitt
- 100: Verstellelement
- 101: atraumatische Kugel
- 102: Fangkorb
- 103: Ösen
- 104: Verstellelement
- 105: distales Ende
- 110: Fangkorb
- 111: Beschichtung
- 112: Zwischenraum
- 113: Netzartige Struktur
- 120: Einrichtung zum Zerschneiden
- 121: Kugel
- 122: Draht
- 123: Drahtschlinge
- 124: Wendelförmiger Abschnitt
- 125: Schlingenförmiger Abschnitt
- 130: Absaugeinrichtung
- 131: Kanüle
- 132: Kolben
- 133: Zylinderteil
- 134: Abzweigstutzen
- 135: Griffteil
- x: distaler Bereich
- y: proximaler Teilbereich
- a₁: Abstand
- d₁: Gefäßdurchmesser
- d₂: aufgeweiteter Gefäßdurchmesser
- s: Strecke

## Patentansprüche

1. Extraktionsvorrichtung (1) zur Extraktion von Objekten (2), insbesondere Thromben, Fremdkörpern etc., aus Hohlräumen (3) eines menschlichen oder tierischen Körpers mit zumindest einem komprimierbaren und expandierbaren Fangkorb (10, 20, 102, 110) mit einem distalen und einem proximalen Ende (12, 13, 22, 23, 105),
wobei
der Fangkorb (20, 110) asymmetrisch, nämlich einseitig verlängert, ausgebildet ist
**dadurch gekennzeichnet, dass**
zumindest ein drahtartiges flexibles Verstellelement (11, 21, 100, 104) an dem distalen und/oder proximalen Ende (12, 13, 22, 23, 105) so befestigt ist, dass der zumindest eine Fangkorb (10, 20, 102) durch diese gezielt dirigierbar und in seiner Form so veränderbar ist, dass der zumindest eine Fangkorb (20) beim Aufweiten in seiner Längserstreckung verkürzt und beim Reduzieren seines Querschnitts verlängert wird.

2. Extraktionsvorrichtung (1) nach Anspruch 1 mit einem ersten und einem zweiten komprimierbaren und expandierbaren Fangkorb (10, 20, 102, 110), zwischen denen das Objekt (2) einfangbar ist, wobei die Fangkörbe gegeneinander verschiebbar und ineinander ziehbar sind,
wobei
der erste Fangkorb (10) im expandierten Zustand schirmartig und mit flexiblen drahtartigen Verstellelementen zum gezielten Ändern der Form und/oder Position des Fangkorbs so ausgebildet ist, dass der erste Fangkorb (10) beim Aufweiten in seiner Längserstreckung verkürzt und beim Reduzieren seines Querschnitts verlängert wird, und dass das Objekt in diesem einfangbar und in den zweiten Fangkorb (20) hineinziehbar ist.

3. Extraktionsvorrichtung (1) nach Anspruch 2,
**dadurch gekennzeichnet, dass**
der schirmartige erste Fangkorb sich zu dem zweiten Fangkorb hin oder von dem zweiten Fangkorb wegweisend öffnet.

4. Extraktionsvorrichtung (1) nach Anspruch 2 oder 3,
**dadurch gekennzeichnet, dass**
beide Fangkörbe (10, 20, 102, 110) mit zumindest einem Verstellelement-(11, 21, 100) zum gezielten Verstellen der Form und/oder Position der Fangkörbe versehen sind.

5. Extraktionsvorrichtung (1) nach einem der vorstehenden Ansprüche,
**dadurch gekennzeichnet, dass**
das zumindest eine Verstellelement (11, 21, 140, 104) ein oder mehrere dünne Drähte aufweist.

6. Extraktionsvorrichtung (1) nach einem der vorstehenden Ansprüche,
**dadurch gekennzeichnet, dass**
das zumindest eine Verstellelement (11, 21, 100, 104) auf der Außenseite und/oder Innenseite des zumindest einen Fangkorbs (10, 20, 102) angeordnet ist, insbesondere in die Mantelfläche (29) des Fangkorbs zumindest teilweise integriert und/oder dort eingeflochten ist.

7. Extraktionsvorrichtung (1) nach einem der vorstehenden Ansprüche,
**dadurch gekennzeichnet, dass**
das zumindest eine Verstellelement (11, 21, 100, 104) über die ausgestreckte Länge des zumindest einen Fangkorbs hinausragt und insbesondere proximal betätigbar angeordnet ist, insbesondere über einen Handgriff betätigbar ist.

8. Extraktionsvorrichtung (1) nach einem der vorstehenden Ansprüche,
**dadurch gekennzeichnet, dass**
bei Vorsehen eines Verstellelements (11) am proximalen Ende (12) des Fangkorbs (10) dieser asymmetrisch, insbesondere im Bereich der Befestigung des Verstellelements (11) einseitig verlängert ausgebildet und/oder mit einem hakenförmige Element (75) zum Angreifen eines Verstell- und/oder Führungselements versehen ist.

9. Extraktionsvorrichtung (1) nach einem der vorstehenden Ansprüche,
**dadurch gekennzeichnet, dass**
das oder die Verstellelemente (11) verzweigt am Fangkorb (10) befestigt und proximal in Gruppen zusammengeführt sind.

10. Extraktionsvorrichtung (1) nach einem der vorstehenden Ansprüche,
**dadurch gekennzeichnet, dass**
das zumindest eine Verstellelement (11, 21) einteilig mit dem Fangkorb (10, 20) ist.

11. Extraktionsvorrichtung (1) nach einem der vorstehenden Ansprüche,
**dadurch gekennzeichnet, dass**
der Abstand zwischen dem distalen Ende (12) des Fangkorbs (10) und der zumindest einen proximalen Befestigungs- oder Austrittsstelle (18, 19) des zumindest einen Verstellelements (11) für unterschiedliche Ausbildungen des Fangkorbs gleichbleibend Ist.

12. Extraktionsvorrichtung (1) nach einem der vorstehenden Ansprüche,
**dadurch gekennzeichnet, dass**
das proximale Ende (23) des zumindest einen Fangkorbs (20) In einem rohrförmigen Element, insbesondere einem Katheter (40), festlegbar oder festgelegt ist und das oder die Verstellelemente (21) durch das rohrförmigen Element geführt oder führbar sind.

13. Extraktionsvorrichtung (1) nach einem der vorstehenden Ansprüche,
**dadurch gekennzeichnet, dass**
der zumindest eine Fangkorb (20) so ausgebildet ist, dass er beim Aufweiten auf einen größeren Durchmesser (d₂) als den Durchmesser (d₁) des auszuräumenden Hohlraums (3) zum partiellen Aufweiten des Hohlraums expandierbar ist.

14. Extraktionsvorrichtung (1) nach einem der Ansprüche 12 bis 13,
**dadurch gekennzeichnet, dass**
am proximalen Ende (23) des zumindest einen Fangkorbs (20) ein Hülsenelement (24) zum Verstärken der Verbindung zwischen rohrförmigem Element (40) und Fangkorb (20) vorgesehen ist.

15. Extraktionsvorrichtung (1) nach einem der Ansprüche 12 bis 14,
**dadurch gekennzeichnet, dass**
das rohrförmige Element (40) einteilig mit dem zweiten Fangkorb (20) ausgebildet und zumindest teilweise mit einem ein Expandieren und Komprimieren ermöglichenden Schnitt versehen ist.

16. Extraktionsvorrichtung (1) nach einem der vorstehenden Ansprüche,
**dadurch gekennzeichnet, dass**
quer zu der Längserstreckung des zumindest einen Fangkorbs (10) angeordnete Reduzierelemente (60, 61) vorgesehen sind, insbesondere im Bereich der proximalen und/oder distalen Enden (12, 13) des Fangkorbs und/oder im Bereich der zumindest einen proximalen Befestigungs- oder Austrittsstelle (18, 19) des zumindest einen Verstellelements (11), insbesondere die Reduziereleinente (60, 61) Schlingen sind.

17. Extraktionsvorrichtung (1) nach einem der vorstehenden Ansprüche,
**dadurch gekennzeichnet, dass**
das oder die Verstellelemente (11, 21, 100, 104) in zumindest einem rohrförmigen Element (40), insbesondere einem Katheter, festgelegt oder beweglich geführt sind.

18. Extraktionsvorrichtung (1) nach einem der vorstehenden Ansprüche,
**dadurch gekennzeichnet, dass**
eine Führungskanüle (14, 74) vorgesehen ist, die an dem distalen Ende (12) des oder des ersten Fangkorbs (10) befestigt ist.

19. Extraktionsvorrichtung (1) nach Anspruch 18,
**dadurch gekennzeichnet, dass**
die Führungskanüle (14, 74) und/oder das oder die rohrförmigen Elemente (40) aus einem biegsamen Material, insbesondere einem Metall, einer Metalllegierung, einem Kunststoff oder einem anderen biegsamen Material oder einer Materialkombination, insbesondere aus Nitinol besteht oder bestehen.

20. Extraktionsvorrichtung (1) nach einem der vorstehenden Ansprüche,
**dadurch gekennzeichnet, dass**
ein Schleusenelement (50) vorgesehen ist, das einen solchen Innendurchmesser aufweist, dass dadurch der zumindest eine Fangkorb (10, 20, 102), eine Führungskanüle (14, 74) und/oder rohrförmige Elemente (40) und das oder die Verstellelemente (11, 21, 100, 104) durchführbar sind.

21. Extraktionsvorrichtung (1) nach Anspruch 20,
**dadurch gekennzeichnet, dass**
das Schleusenelement (50) aus einem stabilen und zumindest teilweise biegsamen Material besteht, insbesondere aus einem Kunststoff, Metall,
einer Metalllegierung, insbesondere Nitinol, insbesondere einem dünnwandigen Nitinol-Rohr.

22. Extraktionsvorrichtung (1) nach einem der vorstehenden Ansprüche,
**dadurch gekennzeichnet, dass**
zumindest ein Fangkorb (10, 20, 102) aus einem Geflecht und/oder Gewebe und/oder Gelege besteht, insbesondere einem Drahtgeflecht und/oder Drahtgewebe und/oder Drahtgelege und/oder zumindest ein Fangkorb (10, 20, 102) aus einem zumindest über einen Teil seiner Länge geschlitzten Rohr (90) besteht und/oder mit einer Beschichtung (111) versehen ist.

23. Extraktionsvorrichtung (1) nach einem der Ansprüche 3 bis 22,
**dadurch gekennzeichnet, dass**
das zumindest eine Verstellelement (11, 21, 100, 104) aus einem Teil eines Geflechts, Gewebes oder Geleges ausgebildet ist.

24. Extraktionsvorrichtung (1) nach einem der vorstehenden Ansprüche,
**dadurch gekennzeichnet, dass**
der zumindest eine Fangkorb (10, 20, 102, 110) aus einem biokompatiblen Material, insbesondere einem Metall oder einer Metalllegierung,
insbesondere einem Edelstahl oder Nitinol besteht und/oder das Material des zumindest einen Fangkorbs (10, 20, 102, 110) mit einem Material beschichtet ist, insbesondere eine biokompatible Oberflächenbeschichtung, Heparin, eine Karbonisierung von Nitinol, eine nanotechnologische Beschichtung, röntgendichte Partikel, eine einen Wirkstoff freisetzende Beschichtung, eine insbesondere mikroporöse biotechnologische oder eine andere Beschichtung aufweist.

25. Extraktionsvorrichtung (1) nach Anspruch 24
**dadurch gekennzeichnet, dass**
das Material des zumindest einen Fangkorbs (20) in zumindest einem Teilbereich chemisch und/oder mechanisch behandelt, insbesondere geätzt, elektropoliert, mikrogeschliffen oder anderweitig behandelt ist.

26. Extraktionsvorrichtung (1) nach einem der vorstehenden Ansprüche,
**dadurch gekennzeichnet, dass**
ein Führungsdraht (30) und/oder Innenmandrin vorgesehen ist, entlang dem der zumindest eine oder die beiden Fangkörbe (10, 20, 102, 110) verschiebbar und/oder in den Hohlraum (3) einführbar sind.

27. Extraktionsvorrichtung (1) nach einem der vorstehenden Ansprüche,
**dadurch gekennzeichnet, dass**
die Extraktionsvorrichtung (1) in Verbindung mit einem Endoskop mit oder ohne Vorsehen des Schleusenelements (50) verwendbar ist.

28. Extraktionsvorrichtung (1) nach einem der vorstehenden Ansprüche,
**dadurch gekennzeichnet dass**
eine Einrichtung (120) zum Zerschneiden oder Zerteilen von Objekten (2) vorgesehen ist, insbesondere ein mit einer Materialverdickung, insbesondere einer Kugel (121), einem wendelförmigen Abschnitt (124), einem schlingenförmigen Abschnitt (125), einer Kombination von diesen oder einer anderen Art von Materialverdickung, versehener Draht (122), der innerhalb des Fangkorbs beweglich anordbar oder angeordnet ist und/oder ein mit einem Stent oder dergleichen Element versehener Ballonkatheter.

29. Extraktionsvorrichtung (1) nach einem der vorstehenden Ansprüche,
**dadurch gekennzeichnet, dass**
eine Absaugeinrichtung (130) zum Absaugen von Objekten oder Objektteilen vorgesehen ist, insbesondere eine Kanüle (131) oder dergleichen rohrförmige Einrichtung umfasst, die in den Bereich des Fangkorbs führbar und mit einem Unterdruck beaufschlagbar ist.

## Claims

1. Device (1) for extracting objects (2), especially thrombi, foreign bodies etc., from cavities (3) in a human or animal body, comprising at least one compressible and expandable trapping net (10, 20, 102, 110) with a distal and a proximal end (12, 13, 22, 23, 105),
wherein
the trapping net (20, 110) is of asymmetric design, i.e. longer on one side,
**characterized in that**
at least one wire-like, flexible adjusting element (11, 21, 100, 104) is attached to the distal and/or proximal end (12, 13, 22, 23, 105) so that the at least one trapping net (10, 20, 102) can be specifically directed by said elements and can change its shape in such a way that the at least one trapping net (20) is shortened lengthwise on widening and lengthened on reduction of its cross-section.

2. Extraction device (1) according to Claim 1 comprising a first and a second compressible and expandable trapping net (10, 20, 102, 110) between which the object (2) can be trapped, it being possible to move the trapping nets towards one another and pull them into one another,
wherein
the first trapping net (10) is like an umbrella in the expanded state and, with flexible, wire-like adjusting elements for specifically changing the shape and/or position of the trapping net, is designed so that it is shortened lengthwise on widening and lengthened on reduction of its cross-section, and so that the object can be trapped in said first trapping net (10) and pulled into the second trapping net (20).

3. Extraction device (1) according to Claim 2,
**characterized in that**
the first, umbrella-like trapping net opens towards the second trapping net or away from the second trapping net.

4. Extraction device (1) according to Claim 2 or 3,
**characterized in that**
both trapping nets (10, 20, 102, 110) are provided with at least one adjusting element (11, 21, 100) for specifically adjusting the shape and/or position of the trapping nets.

5. Extraction device (1) according to one of the preceding claims,
**characterized in that**
the at least one adjusting element (11, 21, 100, 104) has one or more thin wires.

6. Extraction device (1) according to one of the preceding claims,
**characterized in that**
the at least one adjusting element (11, 21, 100, 104) is arranged on the outside and/or inside of the at least one trapping net (10, 20, 102) and, in particular, is at least partially integrated into the envelope (29) of the trapping net and/or is plaited into it.

7. Extraction device (1) according to one of the preceding claims,
**characterized in that**
the at least one adjusting element (11, 21, 100, 104) protrudes beyond the extended length of the at least one trapping net and, in particular, is arranged so that it can be actuated at the proximal end, especially by means of a handle.

8. Extraction device (1) according to one of the preceding claims,
**characterized in that**
when an adjusting element (11) is provided at the proximal end (12) of the trapping net (10), the latter is of asymmetric design and, in particular, is longer on one side in the region where the adjusting element (11) is attached, and/or is provided with a hook-shaped element (75) for gripping an adjusting and/or guiding element.

9. Extraction device (1) according to one of the preceding claims,
**characterized in that**
the adjusting element(s) (11) are attached as a branch to the trapping net (10) and grouped together at the proximal end.

10. Extraction device (1) according to one of the preceding claims,
**characterized in that**
the at least one adjusting element (11, 21) is in one piece with the trapping net (10, 20).

11. Extraction device (1) according to one of the preceding claims,
**characterized in that**
the distance between the distal end (12) of the trapping net (10) and the at least one proximal attachment or exit point (18, 19) on the at least one adjusting element (11) is the same for different designs of the trapping net.

12. Extraction device (1) according to one of the preceding claims,
**characterized in that**
the proximal end (23) of the at least one trapping net (20) can be, or is, fixed in a tubular element, especially a catheter (40), and the adjusting element(s) (21) are, or can be, guided through the tubular element.

13. Extraction device (1) according to one of the preceding claims,
**characterized in that**
the at least one trapping net (20) is designed so that it can expand to widen part of the cavity (3) to be cleared, by widening to a diameter (d₂) greater than the diameter (d₁) of said cavity.

14. Extraction device (1) according to Claim 12 or 13,
**characterized in that**,
at the proximal end (23) of the at least one trapping net (20), a sheathing element (24) is provided for strengthening the joint between the tubular element (40) and the trapping net (20).

15. Extraction device (1) according to one of Claims 12 to 14,
**characterized in that**
the tubular element (40) is designed in one piece with the second trapping net (20) and at least part of it is provided with a cut to allow expansion and compression.

16. Extraction device (1) according to one of the preceding claims,
**characterized in that**
provision is made for reducing elements (60, 61) transverse to the length of the at least one trapping net (10), especially in the region of the proximal and/or distal ends (12, 13) of the trapping net and/or in the region of the at least one proximal attachment or exit point (18, 19) on the at least one adjusting element (11), said reducing elements (60, 61) being loops in particular.

17. Extraction device (1) according to one of the preceding claims,
**characterized in that**
the adjusting element(s) (11, 21, 100, 104) are fixed or movably guided in at least one tubular element (40), especially a catheter.

18. Extraction device (1) according to one of the preceding claims,
**characterized in that**
provision is made for a guide cannula (14, 74) attached to the distal end (12) of the, or of the first, trapping net (10).

19. Extraction device (1) according to Claim 18,
**characterized in that**
the guide cannula (14, 74) and/or the tubular element(s) (40) are made of a flexible material, especially a metal, a metal alloy, a plastic or some other flexible material or a combination of materials, especially nitinol.

20. Extraction device (1) according to one of the preceding claims,
**characterized in that**
provision is made for a channelling element (50) whose internal diameter is such that the at least one trapping net (10, 20, 102), a guide cannula (14, 74) and/or tubular elements (40) and the adjusting element(s) (11, 21, 100, 104) can be passed through it.

21. Extraction device (1) according to Claim 20,
**characterized in that**
the channelling element (50) is made of a robust and at least partially flexible material, especially a plastic, metal or metal alloy, particularly nitinol, and consists especially of a thin-walled nitinol tube.

22. Extraction device (1) according to one of the preceding claims,
**characterized in that**
at least one trapping net (10, 20, 102) consists of a plaited and/or woven and/or non-crimp fabric,
especially a plaited and/or woven and/or non-crimp wire fabric, and/or at least one trapping net (10, 20, 102) consists of a tube (90) slit over at least part of its length, and/or is provided with a coating (111).

23. Extraction device (1) according to one of Claims 3 to 22,
**characterized in that**
the at least one adjusting element (11, 21, 100, 104) is formed of part of a plaited, woven or non-crimp fabric.

24. Extraction device (1) according to one of the preceding claims,
**characterized in that**
the at least one trapping net (10, 20, 102, 110) is made of a biocompatible material, especially a metal or metal alloy and particularly stainless steel or nitinol, and/or the material of the at least one trapping net (10, 20, 102, 110) is coated with a material and in particular has a biocompatible surface coating, heparin, a carbonization product of nitinol, a nanotechnological coating, particles impervious to X-rays, a coating that releases an active ingredient, a particularly microporous biotechnological coating or some other coating.

25. Extraction device (1) according to Claim 24,
**characterized in that**
at least part of the material of the at least one trapping net (20) has been chemically or mechanically treated, especially by etching, electropolishing, microgrinding or some other process.

26. Extraction device (1) according to one of the preceding claims,
**characterized in that**
provision is made for a guide wire (30) and/or inner mandrel along which the at least one trapping net (10, 20, 102, 110), or both of them, can be moved and/or introduced into the cavity (3).

27. Extraction device (1) according to one of the preceding claims,
**characterized in that**
it can be used in combination with an endoscope, with or without provision of the channelling element (50).

28. Extraction device (1) according to one of the preceding claims,
**characterized in that**
provision is made for a device (120) for cutting or breaking up objects (2), especially a wire (122) provided with a thicker section of material, particularly a ball (121), a spiral section (124), a loop-shaped section (125), a combination of these or some other type of thicker section of material, which can be, or is, arranged so as to move inside the trapping net, and/or a balloon catheter provided with a stent or similar element.

29. Extraction device (1) according to one of the preceding claims,
**characterized in that**
provision is made for a device (130) for sucking up objects or parts of objects, especially comprising a cannula (131) or similar tubular device which can be guided into the region of the trapping net and partially evacuated.

## Revendications

1. Dispositif d'extraction (1) pour l'extraction d'objets (2), en particulier des thrombus, des corps étrangers (3), etc. de cavités (3) d'un corps humain ou animal, avec au moins un panier récepteur (10, 20, 102, 110) compressible et expansible doté d'une extrémité distale et d'une extrémité proximale (12, 13, 22, 23, 105),
sachant que
le panier récepteur (20, 110) est réalisé asymétrique, à savoir rallongé d'un côté,
au moins un élément de réglage (11, 21, 100, 104) flexible de type fil métallique est fixé sur l'extrémité distale et/ou proximale (12, 13, 22, 23, 105) de sorte qu'au moins un panier récepteur (10, 20, 102) puisse être dirigé de manière ciblée par celui-ci et sa forme puisse être modifiée si bien qu'au moins un panier récepteur (20) soit raccourci lors de l'élargissement dans son étendue longitudinale et soit rallongé lors de la réduction de sa section transversale.

2. Dispositif d'extraction (1) selon la revendication 1 doté d'un premier et d'un second paniers récepteurs (10, 20, 102, 110) compressibles et expansibles, entre lesquels l'objet (2) peut être piégé, sachant que les paniers récepteurs peuvent être déplacés l'un contre l'autre et engagés l'un dans l'autre, sachant que le premier panier récepteur (10) est réalisé à l'état expansé comme un parapluie et avec des éléments de réglage flexibles de type fil métallique pour la modification ciblée de la forme et/ou de la position du panier récepteur de sorte que le premier panier récepteur (20) soit raccourci lors de l'élargissement dans son étendue longitudinale et soit rallongé lors de la réduction de sa section transversale, et que l'objet puisse être piégé dans celui-ci et engagé dans le second panier récepteur (20).

3. Dispositif d'extraction (1) selon la revendication 2, **caractérisé en ce que** le premier panier récepteur de type parapluie s'ouvre vers ou loin du second panier récepteur.

4. Dispositif d'extraction (1) selon la revendication 2 ou 3, **caractérisé en ce que** les deux paniers récepteurs (10, 20, 102, 110) sont pourvus d'au moins un élément de réglage (11, 21, 100) servant au réglage ciblé de la forme et/ou de la position des paniers récepteurs.

5. Dispositif d'extraction (1) selon l'une quelconque des revendications précédentes, **caractérisé en ce qu'**au moins un élément de réglage (11, 21, 100, 104) présente un ou plusieurs fils métalliques minces.

6. Dispositif d'extraction (1) selon l'une quelconque des revendications précédentes, **caractérisé en ce qu'**au moins un élément de réglage (11, 21, 100, 104) est disposé sur le côté extérieur et/ou côté intérieur d'au moins un panier récepteur (10, 20, 102), en particulier est intégré au moins en partie dans la surface enveloppe (29) du panier récepteur et/ou y est entrelacé.

7. Dispositif d'extraction (1) selon l'une quelconque des revendications précédentes, **caractérisé en ce qu'**au moins un élément de réglage (11, 21, 100, 104) dépasse de la longueur étirée d'au moins un panier récepteur et est disposé de sorte à pouvoir être actionné de manière proximale, en particulier peut être actionné par une poignée.

8. Dispositif d'extraction (1) selon l'une quelconque des revendications précédentes, **caractérisé en ce que** lors de la prévoyance d'un élément de réglage (11) sur l'extrémité proximale (12) du panier récepteur (10), celui-ci est réalisé de manière asymétrique, en particulier rallongé d'un côté dans la zone de la fixation de l'élément de réglage (11) et/ou est pourvu d'un élément (75) en forme de crochet pour saisir un élément de réglage et/ou de guidage.

9. Dispositif d'extraction (1) selon l'une quelconque des revendications précédentes, **caractérisé en ce que** le ou les éléments de réglage (11) sont fixés par dérivation sur le panier récepteur (10) et sont rassemblés de manière proximale en groupes.

10. Dispositif d'extraction (1) selon l'une quelconque des revendications précédentes, **caractérisé en ce qu'**au moins un élément de réglage (11, 21) forme une seule partie avec le panier récepteur (10, 20).

11. Dispositif d'extraction (1) selon l'une quelconque des revendications précédentes, **caractérisé en ce que** la distance entre l'extrémité distale (12) du panier récepteur (10) et au moins un point de fixation ou de sortie proximale (18, 19) d'au moins un élément de réglage (11) est constante pour différentes réalisations du panier récepteur.

12. Dispositif d'extraction (1) selon l'une quelconque des revendications précédentes, **caractérisé en ce que** l'extrémité proximale (23) d'au moins un panier récepteur (20) est ou peut être fixée dans un élément tubulaire, en particulier un cathéter (40) et le ou les éléments de réglage (21) traversent ou peuvent traverser l'élément tubulaire.

13. Dispositif d'extraction (1) selon l'une quelconque des revendications précédentes, **caractérisé en ce qu'**au moins un panier récepteur (20) est réalisé de sorte qu'il puisse être expansé lors de l'élargissement à un diamètre supérieur (d₂) au diamètre (d₁) de la cavité à vider pour son élargissement partiel.

14. Dispositif d'extraction (1) selon l'une quelconque des revendications 12 à 13, **caractérisé en ce qu'**un élément de douille (24) est prévu sur l'extrémité proximale (23) d'au moins un panier récepteur (20) pour le renforcement de la liaison entre l'élément tubulaire (40) et le panier récepteur (20).

15. Dispositif d'extraction (1) selon l'une quelconque des revendications 12 à 14, **caractérisé en ce que** l'élément tubulaire (40) est réalisé d'un seul tenant avec le second panier récepteur (20) et est pourvu au moins en partie d'une entaille permettant une expansion et une compression.

16. Dispositif d'extraction (1) selon l'une quelconque des revendications précédentes, **caractérisé en ce que** des éléments de réduction (60, 61) disposés transversalement à l'étendue longitudinale d'au moins un panier récepteur (10) sont prévus, en particulier dans la zone des extrémités proximales et/ou distales (12, 13) du panier récepteur et/ou dans la zone d'au moins un point de fixation ou de sortie proximale (18, 19) d'au moins un élément de réglage (11), en particulier les éléments de réduction (60, 61) sont des boucles.

17. Dispositif d'extraction (1) selon l'une quelconque des revendications précédentes, **caractérisé en ce que** le ou les éléments de réglage (11, 21, 100, 104) sont fixés ou guidés de manière mobile dans au moins un élément tubulaire (40), en particulier un cathéter.

18. Dispositif d'extraction (1) selon l'une quelconque des revendications précédentes, **caractérisé en ce qu'**une canule de guidage (14, 74) est prévue, laquelle est fixée sur l'extrémité distale (12) du premier panier récepteur (10).

19. Dispositif d'extraction (1) selon la revendication 18, **caractérisé en ce que** la canule de guidage (14, 74) et/ou le ou les éléments tubulaires (40) se composent d'un matériau flexible, en particulier un métal, un alliage métallique, un plastique ou un autre matériau flexible ou une combinaison de matériaux, en particulier de nitinol.

20. Dispositif d'extraction (1) selon l'une quelconque des revendications précédentes, **caractérisé en ce qu'**un élément de sas (50) est prévu, lequel présente un tel diamètre intérieur qu'au moins un panier récepteur (10, 20, 102), une canule de guidage (14, 74) et/ou des éléments tubulaires (40) et le ou les éléments de réglage (11, 21, 100, 104) peuvent le traverser.

21. Dispositif d'extraction (1) selon la revendication 20, **caractérisé en ce que** l'élément de sas (50) se compose d'un matériau stable et au moins en partie flexible, en particulier d'un plastique, d'un métal, d'un alliage métallique, en particulier du nitinol, en particulier un tube en nitinol à parois minces.

22. Dispositif d'extraction (1) selon l'une quelconque des revendications précédentes, **caractérisé en ce qu'**au moins un panier récepteur (10, 20, 102) se compose d'un tressage et/ou d'un tissage et/ou d'un non tissé, en particulier d'un tressage de fils métalliques et/ou d'un tissage métallique et/ou d'un non tissé métallique et/ou au moins un panier récepteur (10, 20, 102) se compose d'un tube (90) fendu au moins sur une partie de sa longueur et/ou est pourvu d'un revêtement (111).

23. Dispositif d'extraction (1) selon l'une quelconque des revendications 3 à 22, **caractérisé en ce qu'**au moins un élément de réglage (11, 21, 100, 104) est composé d'une partie d'un tressage, d'un tissage ou d'un non tissé.

24. Dispositif d'extraction (1) selon l'une quelconque des revendications précédentes, **caractérisé en ce qu'**au moins un panier récepteur (10, 20, 102, 110) se compose d'un matériau biocompatible, en particulier d'un métal ou d'un alliage métallique, en particulier d'un acier spécial ou de nitinol et/ou le matériau d'au moins un panier récepteur (10, 20, 102, 110) est revêtu d'un matériau, en particulier présente un revêtement superficiel biocompatible, de l'héparine, une carbonisation de nitinol, un revêtement nanotechnologique, des particules étanches aux rayons X, un revêtement libérant une substance active, un revêtement biotechnologique en particulier microporeux ou un autre revêtement.

25. Dispositif d'extraction (1) selon la revendication 24, **caractérisé en ce que** le matériau d'au moins un panier récepteur (20) est traité chimiquement et/ou mécaniquement dans au moins une zone partielle, en particulier est corrodé, électropoli, micromeulé ou traité autrement.

26. Dispositif d'extraction (1) selon l'une quelconque des revendications précédentes, **caractérisé en ce qu'**un fil conducteur (30) et/ou un mandrin interne est prévu, le long duquel au moins un ou les deux paniers récepteurs (10, 20, 102, 110) peuvent être déplacés et/ou introduits dans la cavité (3).

27. Dispositif d'extraction (1) selon l'une quelconque des revendications précédentes, **caractérisé en ce que** le dispositif d'extraction (1) peut être utilisé en liaison avec un endoscope avec ou sans élément de sas (50) prévu.

28. Dispositif d'extraction (1) selon l'une quelconque des revendications précédentes, **caractérisé en ce qu'**un dispositif (120) pour découper ou décomposer des objets (2) est prévu, en particulier un fil métallique (122) pourvu d'un épaississement de matériau, en particulier une bille (121), une section hélicoïdale (124), une section en forme de boucle (125), une combinaison de celles-ci ou un autre type d'épaississement de matériau, lequel fil est ou peut être disposé de manière mobile dans le panier récepteur et/ou une sonde à ballon pourvue d'un stent ou élément similaire.

29. Dispositif d'extraction (1) selon l'une quelconque des revendications précédentes, **caractérisé en ce qu'**un dispositif d'aspiration (130) est prévu pour l'aspiration d'objets ou de parties d'objet, comporte en particulier une canule (131) ou un dispositif tubulaire similaire qui peut être guidé dans la zone du panier récepteur et peut être alimenté en une dépression.
